# EUROPEAN PATENT SPECIFICATION

(11) **EP 0 364 344 B1**
(45) Date of publication and mention of the grant of the patent: **06.05.1998**
(21) Application number: 89402763.0
(22) Date of filing: 06.10.1989
(51) Int. Cl.: C07K 5/02, C07K 5/06, C07K 5/08, C07K 5/10, C07C 237/20, A61K 38/55

(54) **Novel peptidase inhibitors**
Peptidase-Hemmer
Inhibiteurs de peptidases

(30) Priority: 07.10.1988 US 254762
(43) Date of publication of application: 18.04.1990
(73) Proprietor: MERRELL PHARMACEUTICALS INC., Cincinnati, Ohio 45215-6300 (US)
(72) Inventor: Bey, Philippe, Cincinnati Ohio 45242 (US); Angelastro, Michael, Loveland Ohio 45140 (US); Mehdi, Shujaath, Cincinnati Ohio 45213 (US)
(74) Representative: Gillard, Marie-Louise

(56) References cited:
- EP-A- 0 133 225
- FR-A- 2 537 131
- CHEMICAL ABSTRACTS vol. 99, no. 21, 21st November 1983, pages 122-123, 170031x, Columbus, Ohio, US

## Description

This invention relates to protease enzyme inhibitors useful for a variety of physiological end-use applications.

In its broad aspects, this invention relates to analogs of peptidase substrates in which the nitrogen atom of the scissile amide group of the substrate peptide has been replaced by H, or a substituted malonyl moiety. These analogs of the peptidase substrates provide specific enzyme inhibitors for a variety of proteases, the inhibition of which will have useful physiological consequences in a variety of disease states.

Document EP-A-133 225 relates to peptide derivatives and their pharmaceutical applications as elastase inhibitors in the treatment of emphyzema and arthritis.

Document in Chemical Abstracts, vol. 99, n° 21, 21 November 1983, p. 122 - 123 relates to the "preparation and properties of oxalyl- and malonyl-bis (methionyl) insulin".

In its more specific aspects, this invention relates to derivatives of certain peptidase substrates which are useful in inhibiting serine-, thio-, and metallo-dependent protease enzymes, the inhibition of which will have useful physiological consequences in a variety of disease states.

Still more specifically, this invention relates to derivatives of peptidase substrates which fall within the following generic groupings characterized according to their active site dependencies. Such generic groupings are:
I. Serine Dependent Enzymes: These include such enzymes as Elastase (human leukocyte), Cathepsin G, Thrombin, Plasmin, C-1 Esterase, C-3 Convertase, Urokinase, Plasminogen Activator, Acrosin, β-Lactamase, D-Alanine-D-Alanine Carboxypeptidase, Chymotrypsin, Trypsin and Kallikreins.
II. Thiol Dependent Enzymes: Cathepsin B and Calpain.
III.Metallo Dependent Enzymes: These include Enkephalinase, Pseudomonas Elastase and Leucine Aminopeptidase.

The contemplated peptidase inhibitors of the foregoing enzymes are selected from the generic formula 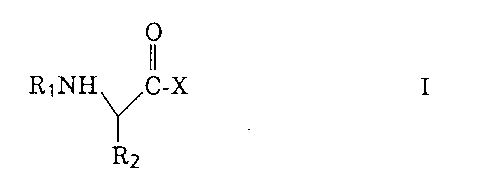 the hydrates, isosteres or the pharmaceutically acceptable salts thereof wherein X is 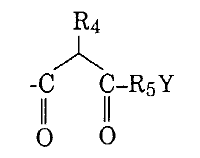
- R₁: is hydrogen, an amino protecting group selected from Group K,an α-amino acid or a peptide comprised of a number of α-amino acid building blocks, said α-amino acid or peptide optionally bearing on its terminal nitrogen atom an amino protecting group selected from Group K,
- R₂: is the "R group" residue of the α-amino acid responsible for directing the inhibitor to the active site of the enzyme or is -A-SiR₇R₈R₉, C₁₋₁₀ alkyl, aralkyl or aryl with R₇, R₈ and R₉, each being selected from C₁₋₁₀ alkyl, aralkyl or aryl and A is a C₁₋₆ alkylene,
- R₄: is the specific R-group residue of the α-amino acid for that peptidase substrate analog,
- R₅: is an α-amino acid or peptide comprised of α-amino acids or is deleted,
- Y: is NHR₃ or OR₃ with R₃ being H, C₁₋₇ alkyl, benzyl or phenethyl.

Unless otherwise stated the α-amino acids of the foregoing peptidase substrates are preferably in their L-configuration. A compound of this invention may be in free form, e.g. amphoteric form, or a salt form, e.g., acid addition or anionic salt. A compound may be converted into its salt or base form in an art-known manner, one from another. Preferred salts are trifluoroacetate, hydrochloride, sodium, potassium or ammonium salts, although the scope of salts embraced herein is not limited thereto, the scope being extended to include all of the salts known to be used in the art of peptide chemistry.

As used herein the term "alkyl" includes the straight, branched-chain and cyclized manifestations thereof, particularly such moieties as methyl, ethyl, n-butyl, t-butyl, cyclopropyl, n-propyl, pentyl, cyclopentyl, n-hexyl, cyclohexyl and cyclohexylmethyl. The term "aralkyl" includes those aryl moieties attached to a C₁₋₄ alkylene. The term "aryl" within the definitions of R₂ includes both carbocyclic and heterocyclic moieties. Preferred aralkyl and aryl moieties are phenyl, benzyl, naphthylmethyl, phenethyl, 2-pyridylmethyl, indolyl, pyridyl, indazolyl, furyl and thienyl are preferred. Other carbocyclics are such fused aryl moieties as pentalenyl, indenyl, naphthalenyl, azulenyl, heptalenyl, acenaphthylenyl, fluorenyl, phenalenyl, phenanthrenyl, anthracenyl, acephenanthrylenyl, aceanthrylenyl, triphenylenyl, pyrenyl, chrysenyl and naphthacenyl. In the term "-A-SiR₇R₈R₉" the alkylene moiety (i.e. "A") is a straight or branched-chain C₁₋₇ alkylene moiety separating the "SiR₇R₈R₉" moiety from the carbon atom to which the "-A-SiR₇R₈R₉" radical is attached. Of the R₇, R₈ and R₉ radicals attached to the silicone atom it is preferred that two or three of these radicals be a C₁₋₇ lower alkyl radical (preferably methyl or ethyl) and that when one of them contains an aryl radical it is preferred that that radical be a benzyl radical. It is preferred that the alkylene moiety be methylene. Preferred moieties are trimethylsilylmethyl, triethylsilylmethyl, benzyldiethylsilylmethyl, benzyldimethylsilylmethyl, benzylethylmethylsilylmethyl, dimethyl-(3-pyridylmethyl)silylmethyl, dimethyl-(3-indolylmethyl)silylmethyl, and the like.

Before further defining and/or illustrating the scope of the peptidase substrate inhibitors embraced by Formula I, it may be convenient to state some of the more basic concepts related to peptides. For example, except for proline, all of the α-amino acids found in proteins have, as a common denominator, a free carboxyl group and a free unsubstituted amino group on the α-carbon atom (in proline, since proline's α-amino group is substituted it is really an α-imino acid, but for convenience, it will also be spoken of as an α-amino group). Additionally, each α-amino acid has a characteristic "R-group", the R-group being the side-chain, or residue, attached to the α-carbon atom of the α-amino acid. For example, the R-group residue for glycine is hydrogen, for alanine it is methyl, for valine it would be isopropyl. (Thus, throughout this specification the R₂ and R₄ moiety is the residue R-group for each indicated α-amino acid or is another radical which may be defined for these sites for any given protease inhibitors). For the specific R-groups - or side chains - of the α-amino acids reference to A.L. Lehninger's text on Biochemistry (see particularly Chapter 4) would be helpful.

As a further convenience for defining the scope of the compounds embraced by the generic concept of Formula 1, as well as the sub-generic concepts relating to each of the individual enzymes involved in this invention, various α-amino acids have been classified into a variety of groups which impart similar functional characteristics for each of the specific enzymes to be inhibited by the peptidase substrates of Formula 1. These groups are set forth in Table II and the recognized abbreviations for the α-amino acid blocks are set forth in Table I.

**TABLE I**

| AMINO ACID | SYMBOL |
|---|---|
| Alanine | Ala |
| Arginine | Arg |
| Aspargine | Asn |
| Aspartic acid | Asp |
| Asn + Asp | Asx |
| Cysteine | Cys |
| Glutamine | Gln |
| Glutamic acid | Glu |
| Gln + Glu | Glx |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophan | Trp |
| Tyrosine | Tyr |
| Valine | Val |
| Norvaline | n-Val |
| Norleucine | n-Leu |
| 1-Naphthylalanine | Nal(1) |
| 2-Indolinecarboxylic acid | Ind |
| Sarcosin | Sar |

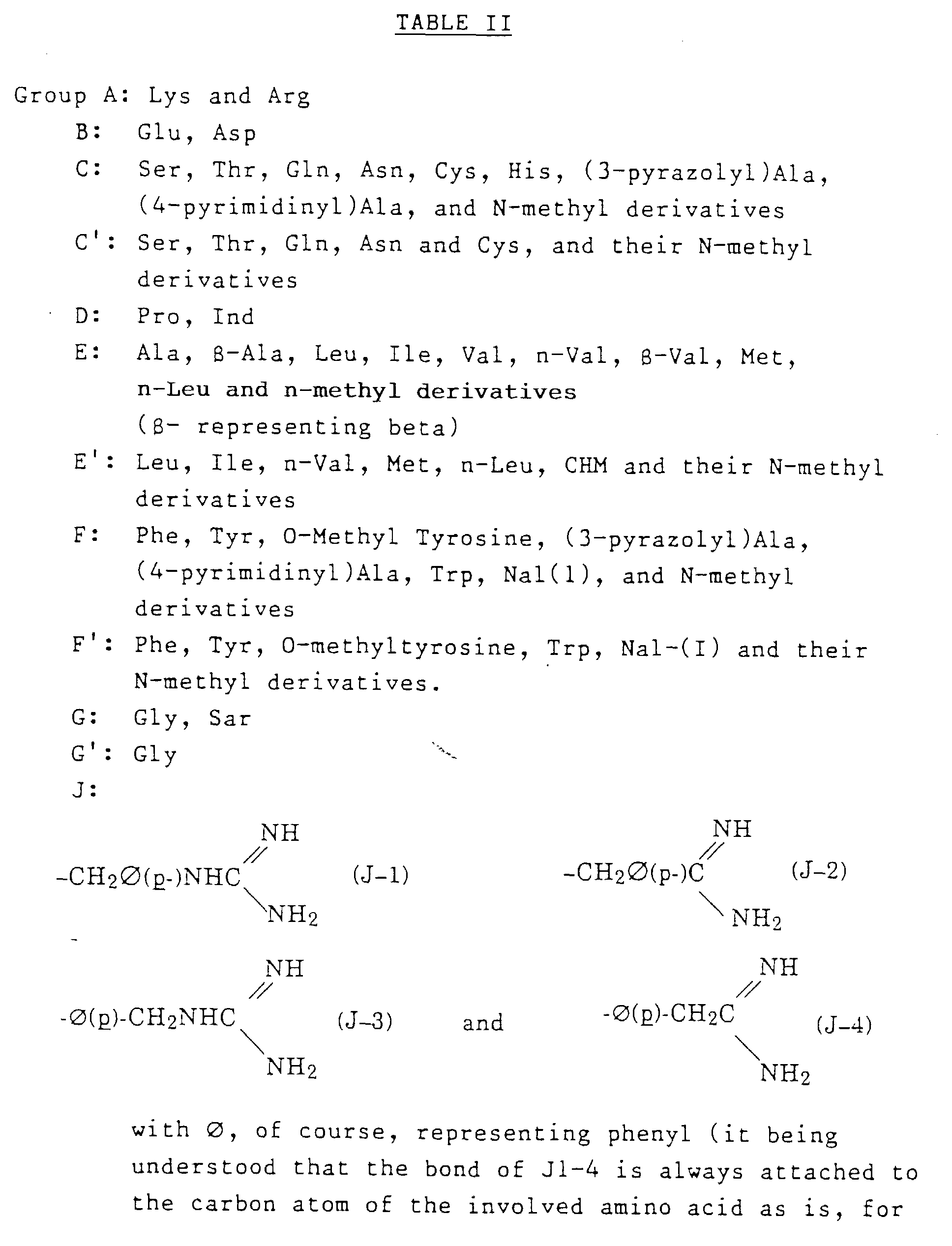

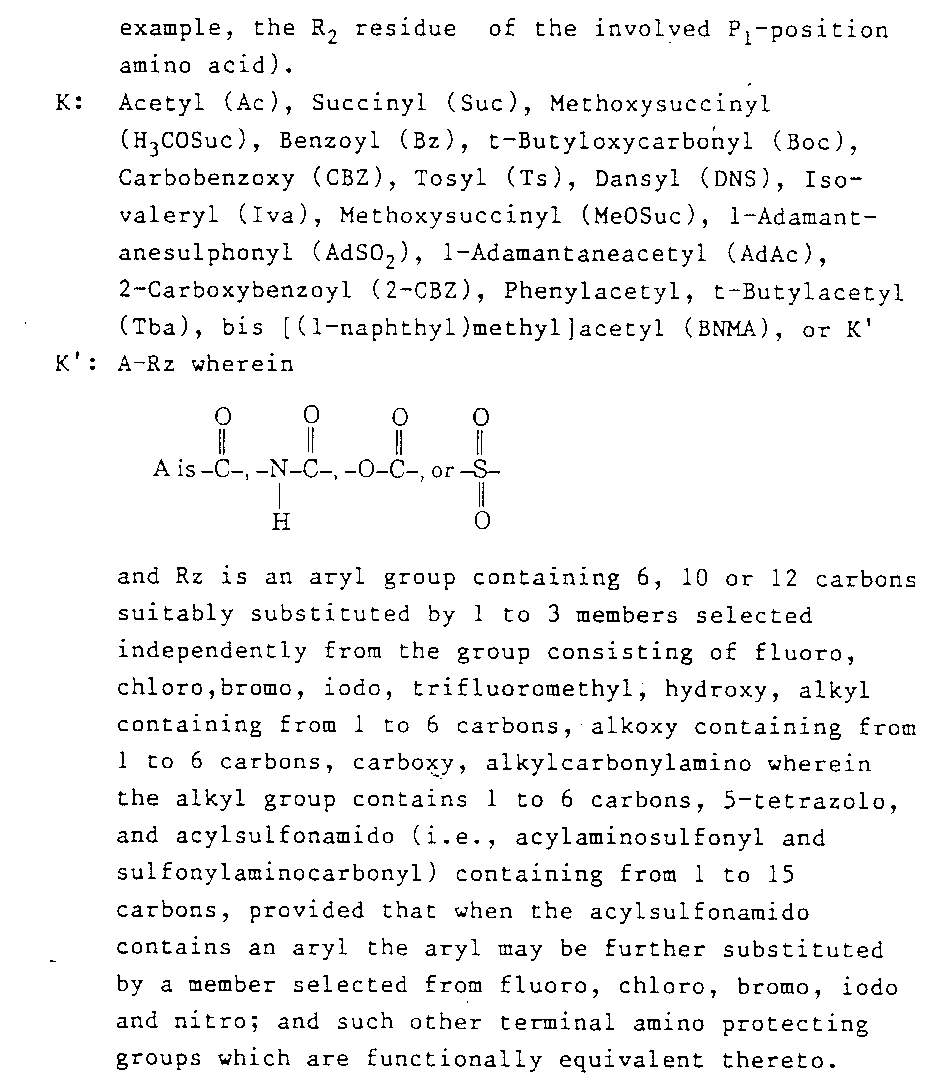

In those instances wherein the normal R-group residue of an α-amino acid contains an -OH radical (e.g. serine, threonine and tyrosine), it is to be understood that such radical can be derivatized. For example, in each of the foregoing instances the -OH radical can be converted to an ether. When so converted, such as for example to their methyl ethers, then such radicals will be referred to as O-methyl Ser, O-methyl Thr and O-methyl Tyr, respectively. These methyl ether radicals may also be depicted as 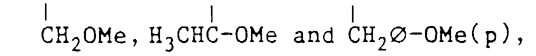 respectively. Similarly, other type derivatives will be analogously represented.

In those instances wherein Group K represents an -A-Rz moiety, it is preferred that A represent -C(=O)- and that Rz represent acylsulfonamido, particularly those wherein the acylsulfonamido contains an aryl moiety (preferably phenyl) substituted by a halogen. The preferred -A-Rz moieties being 4-[(4-chlorophenyl)sulfonylaminocarbonyl]phenylcarbonyl, 4-[(4-bromophenyl)sulfonylaminocarbonyl]phenylcarbonyl and 4-[phenylsulfonylaminocarbonyl]phenylcarbonyl (said moieties being abbreviated as 4-Cl ⌀-SAC-Bz, 4-Br ⌀-SAC-Bz and ⌀-SAC-Bz, respectively).

Quite obviously the modifications to the scissile amide bond of the peptidase substrates of this invention present certain nomenclature difficulties. In order to maintain a general consistency throughout this application the following explanations are offered to obviate any ambiguities relating to the scope and intent of this invention.

In those instances wherein the compounds are defined by the formula 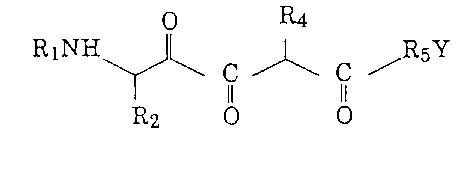 the R₂ moiety is the residue of the α-amino (or other defined moiety) located at the P₁ position, R₁ is either a protecting group moiety from the defined Group K, or is an α-amino acid or peptide moiety (having up to 4 α-amino acids). In defining the R₁ moiety for each specific enzyme, the amino acid or the amino acid of the peptide will be defined according to the P-position it occupies. For example, an R₁ peptide having 2 amino acids will consist of P₂-P₃ moieties, one having 3 amino acids will consist of P₂-P₃-P₄ moieties while one having 4 amino acids will consist of P₂-P₃-P₄-P₅ moieties. In all such instances the terminal nitrogen atom of such moieties may optionally bear a protecting group from the defined K group which, of course, includes the -A-Rz moiety. In defining the specific R₁ moieties for each of the individual protease enzyme inhibitors involved in this invention R₁ will, for example, define a P₂P₃ moiety bearing the Group K protecting group as P₂P₃P_{g}, P_{g} representing a protecting group of Group K on its terminal amine. If the terminal α-amino acid does not bear a protecting group it would then be represented as P₂P₃. In those instances wherein R₅ is a peptide, each α-amino acid will (when appropriate) be numbered sequentially (i.e., R₅₋₁, R₅₋₂, R₅₋₃, etc), and Y will be designated as the terminus of the substrate. In practice, it is preferred that the R₅ moiety contain no more than 3 amino acid units. For example, assume R₁ is a peptide containing two amino acids (Phe and Val) the terminal nitrogen atom of which bears a CBZ moiety, R₂ is the residue of the α-amino acid Arg, R₄ is the residue of the α-amino acid Leu, R₅ is the dipeptide consisting of two amino acids Ser and Val and Y is NHR₃ with R₃ being CH₃. That compound would be written as

CBZ-Phe-Val-Arg[C(O)Leu]-SerValNHCH₃.

The bracketed moiety (i.e.[C(O)Leu]) is used as an alert that the nitrogen atom of the P'₁ α-amino acid has been replaced by a carbonyl function, with Leu being the R₄ residue of that α-amino acid. Of course, it is also to be recognized that the bracketed moiety represents a malonyl moiety but for consistency and convenience it is preferred to designate that moiety as shown. The P'₂ and P'₃ moieties (i.e. SerVal) represent the R₅ moiety, Ser and Val may sometimes be referred to as R₅₋₁ and R₅₋₂, respectively, with NHCH₃ representing the Y group at the terminal portion of the substrate. If R₅ were deleted and Y were OH or OCH₃, the compounds would be written as CBZ-Phe-Val-Arg[C(O)Leu]OH or CBZ-Phe-Val-Arg[C(O)Leu]OCH₃, respectively.

In the light of the foregoing, the compounds of this invention are peptidase inhibitors capable of inhibiting enzymes of the group consisting of Human leukocyte elastase, Cathepsin G, Thrombin, Plasmin, C-1 Esterase, C-3 Convertase, Urokinase, Plasminogen Activator, Acrosin, β-Lactamase, D-Alanine-D-Alanine Carboxypeptidase, Chymotrypsin, Trypsin, Kallikreins, Cathepsin B, Calpain, Retroviral proteases required for replication, Enkephalinase, Pseudomonas elastase and Leucine aminopeptidase and are defined as:

Peptidase inhibitors having the formulae 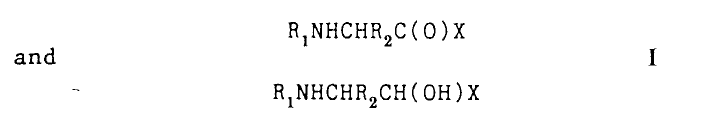 the hydrates, isosteres or the pharmaceutically acceptable salts thereof, wherein
- X: is -C(O)CHR₄C(O)R₅Y,
- R₁: is H, an amino protecting group of Group K, an α-amino acid, a peptide comprised of 2 to 4 α-amino acids, an α-amino acid bearing a Group K protecting group or a peptide comprised of 2 to 4 α-amino acids, the terminal amino acid of which bears a Group K protecting group,
- R₂: is a residue of an α-amino acid, -A-SiR₇R₈R₉, C₁₋₁₀ alkyl, aralkyl or aryl,
- A: is C₁₋₆ alkylene and each of
R₇, R₈ and R₉ being C₁₋₁₀ alkyl, aralkyl or aryl,
- R₄: is a residue of an α-amino acid,
- R₅: is an α-amino acid, a peptide comprised of 2 to 4 α-amino acids or is deleted,
- Y: is NHR₃ or OR₃ with
- R₃: is H, C₁₋₇ alkyl, benzyl or phenethyl,
the said protecting groups, α-amino acids or peptide moieties being selected from Groups A, B, C, D, E, F, G, J, C', E', F', G' and K, said groups being:
- A:: Lys and Arg
- B:: Glu, Asp
- C:: Ser, Thr, Gln, Asn, Cys, His, (3-pyrazolyl)Ala, (4-pyrimidinyl)Ala, and their N-methyl derivatives
- C':: Ser, Thr, Gln, Asn and Cys, and their N-methyl derivatives
- D:: Pro, Ind
- E:: Ala, β-Ala, Leu, Ile, Val, n-Val, β-Val, Met, n-Leu and their methyl derivatives
- E':: Leu, Ile, n-Val, Met, n-Leu, CHM and their N-methyl derivatives
- F:: Phe, Tyr, O-Methyl Tyrosine, (3-pyrazolyl)Ala, (4-pyrimidinyl)Ala, Trp, Nal(1), and their N-methyl derivatives
- F':: Phe, Tyr, O-methyltyrosine, Trp, Nal-(I) and their N-methyl derivatives.
- G:: Gly, Sar
- G':: Gly
- J:: 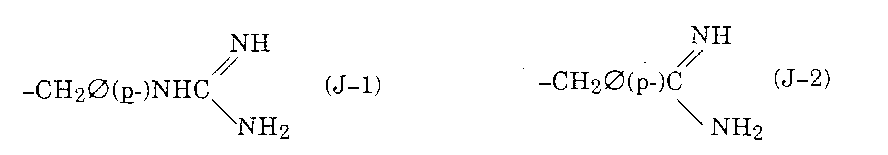 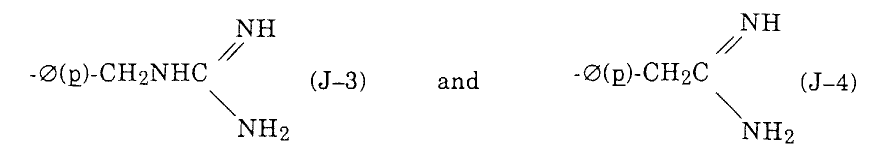
- K:: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA), or K'
- K':: A-Rz wherein 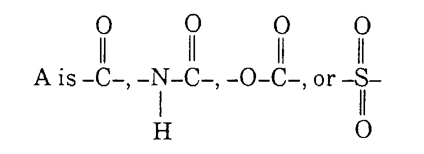 and Rz is an aryl group containing 6, 10 or 12 carbons suitably substituted by 1 to 3 members selected independently from the group consisting of fluoro, chloro,bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolo, and acylsulfonamido containing from 1 to 15 carbons, provided that when the acylsulfonamido contains an aryl the aryl may be further substituted by a member selected from fluoro, chloro, bromo, iodo and nitro.

Compounds of Formula I which are useful as inhibitors of calpain and Cathepsin B are compounds of the formula

R₁NHCHR₂C(O)X **Iu**

wherein X is -C(O)CHR₄C(O)R₅Y,
- R₁: is P₂P₃ or P₂P₃P_{g}, P_{g} being a Group K protecting group, preferably the protecting groups are CBZ, Bz or Ac,
P₂ is an α-amino acid of Groups E or F, preferably Val, Ile, Ala or Pro,
P₃ is an α-amino acid of Groups B, E, F or is deleted, preferably P₃ is deleted or is Ile,
- R₂: is H, a the side chain of α-amino acids of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆ alkylene, preferably R₂ is cyclohexylmethyl, Phe or naphthyl,
- R₃: is C₁₋₆ alkyl, benzyl or phenethyl, preferably C₁₋₆ alkyl,
- R₄: is the residue of an α-amino acid of Groups C, E or H,
- R₅: is deleted, and
- Y: is OR₃ or NHR₃.

By their inhibition of calpain and cathepsin B proteases the compounds of (Iu) will (a) have an effect on cell motility through the extracellular matrix rendering the compounds useful for treating cancer metastases; (b) have long term changes in regulatory proteins (e.g. down-regulation of protein kinase C and breakdown of the cytoskeleton causing secondary effects on platelet activation such as (for enhancing clot formation) leukocyte degranulation (for treating inflammation and immunological diseases, e.g. arthritis, emphysema, multiple sclerosis, and systemic lupus); (c) have a general intracellular proteolysis, particular muscle cells, causing secondary effect on ischemia/reperfusion cell death, thereby rendering the compounds useful for treating stroke and heart attacks; and (d) will aid in blocking the lysis of red blood cells rendering the compounds useful in the treatment of conditions associated with' excessive hemolysis such as in Sickle cell anemia and in kidney dialysis. It is to be expected that the end-use application dose range will be about 0.01 to 10 mg per kg of body weight per day for an effective therapeutic effect.

The preparation of the compounds of this invention may be effected by standard chemical processes analogously known in the art. The processes are depicted in Reaction Schemes A and B and described as follows: 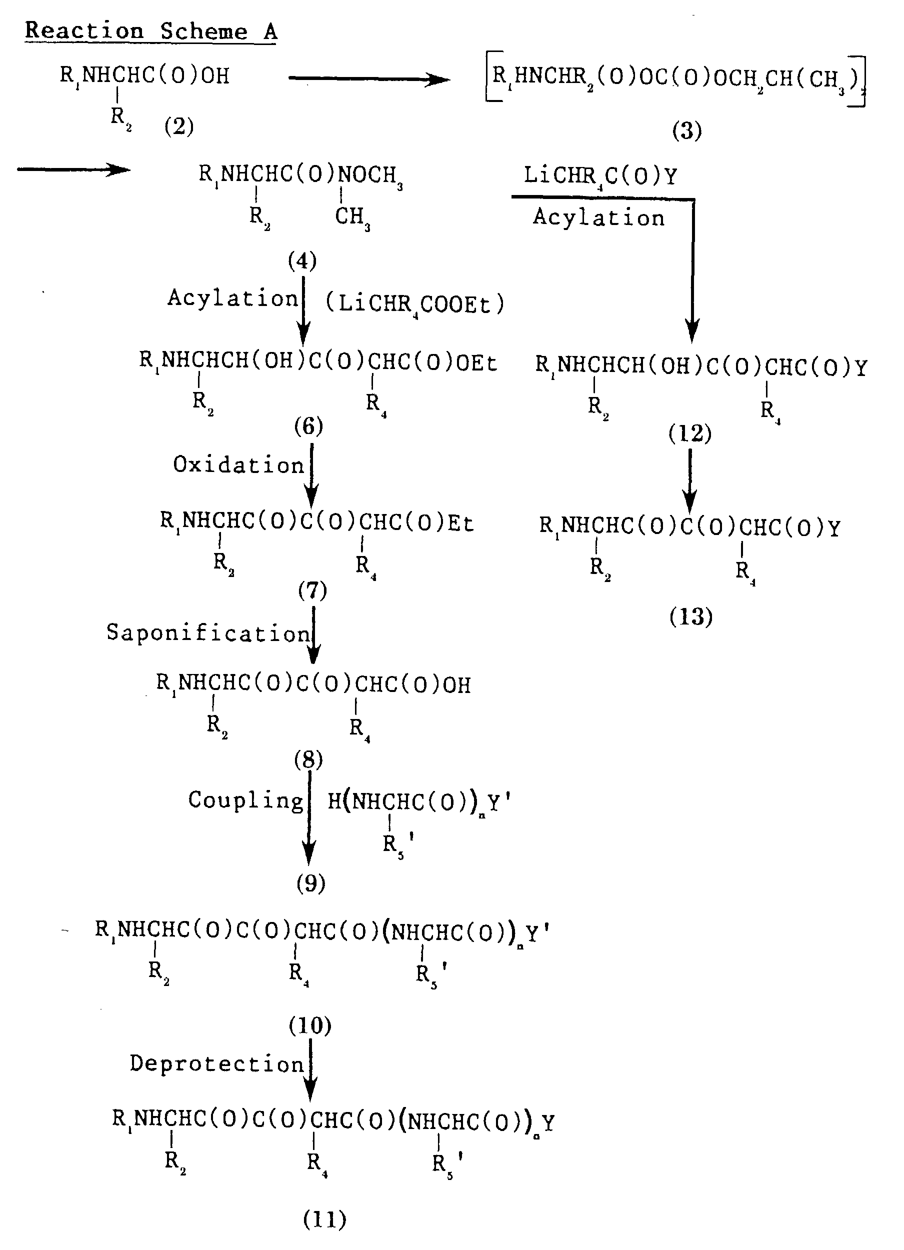 wherein
- Y': is NHR₃, OR₃, P_{g},
- Y: is NHR₃ or OR₃, R₃ being alkyl, benzyl or phenethyl,
- R₅^{'}: is a residue of an amino acid,
- n: is 1 to 4 and R₁, R₂ and R₄ are as previously defined. 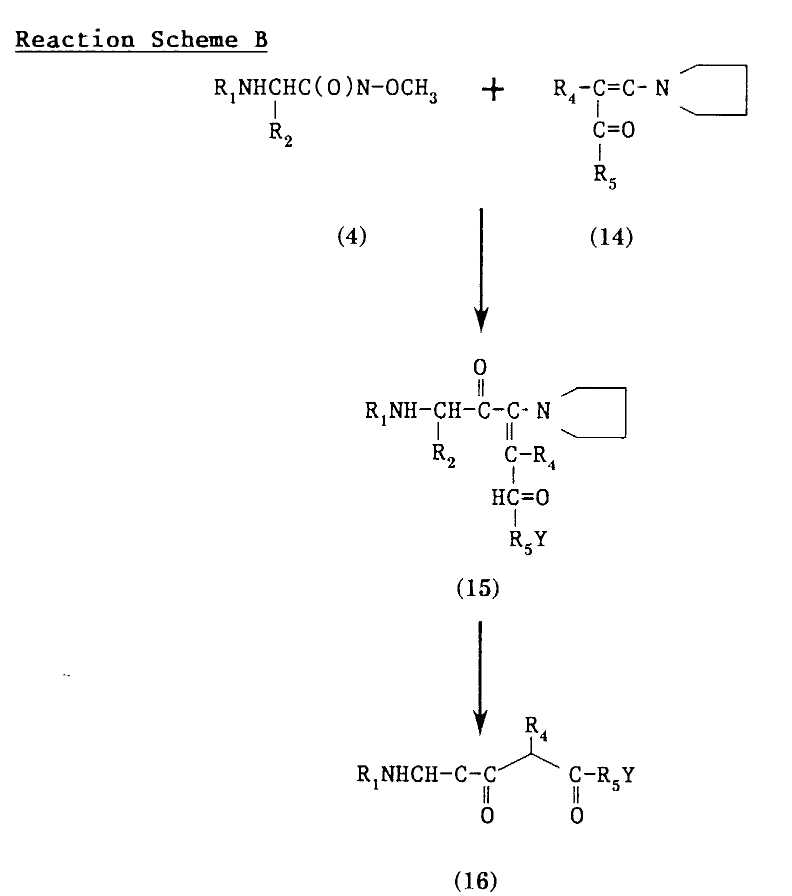
wherein
R₁, R₂, R₄, R₅ and Y are as previously defined. In effecting the processes of the foregoing Reaction Scheme A, the starting materials (2) are subjected to process step (a) which is initiated by anionizing the starting material with a base, preferably N-methyl morpholine, triethylamine (TEA), diisopropylethylamine (DIEA) or other suitable amines. Preferably the anion is formed using excess quantities of the amine, stirring the mixture at about -15°C to 10°C, preferably 0°C. Addition of an equivalent amount of isobutylchloroformate with cooling at about -20°C forms an *in situ* mixed anhydride (3). (Other equivalently functioning peptide coupling agents, such as diethylcyanophosphonate, DCC, BOP reagents, BOP chloride, may be used in place of isobutylchloroformate.) Addition of molar equivalent amounts of N,O-dimethylhydroxylamine to the activated *in situ* intermediate (3) yields a dimethylhydroxamic acid derivative (i.e. an N-methyl-N-methoxy amide) of Formula 4. This coupling step is conducted under an inert atmosphere (argon or nitrogen) under anhydrous conditions.

The so-produced peptidyl, α-hydroxy-N-methyl-N-methoxy amides of Formula (4) are acylated, using the alkyl lithio acetates of Formula (5), by standard acylation conditions such as by reaction of the amides (4) with the alkyl lithio derivatives (5) at about -78°C for about one hour and the resultant reaction mixture is allowed to warm to room temperature, following which the mixture is quenched by its addition to dilute hydrochloric acid to produce the desired intermediates of Formula (6). These hydroxy intermediates are subjected to oxidation procedures such as by use of (1) the Swern oxidation procedure, (2) a modified Jones reaction using pyridinium dichromate, (3) a chromic anhydride-pyridinium complex or (4) with 1,1,1-triacetoxy-2,1-benzoxiodol.

In general the Swern oxidation is effected by reacting about 2 to 10 equivalents of dimethylsulfoxide (DMSO) with about 1 to 6 equivalents of trifluoromethylacetic anhydride [(CF₃CO)₂O] or oxalyl chloride [(COCl)₂], said reactants being dissolved in an inert solvent, e.g., methylene chloride (CH₂Cl₂), said reactor being under an inert atmosphere (e.g., nitrogen or equivalently functioning inert gas) under anhydrous conditions at temperatures of about -80°C to -50°C to form an *in situ* sulfonium adduct to which is added about 1 equivalent of the alcohols of Formula (6). Preferably, the alcohols are dissolved in an inert solvent, e.g., CH₂Cl₂ or minimum amounts of DMSO, and the reaction mixture is allowed to warm to about -50°C (for about 10-20 minutes) and then the reaction is completed by adding about 3 to 10 equivalents of a tertiary amine, e.g., triethylamine, N-methyl morpholine, etc. Following oxidation the desired intermediates (7) are isolated and are ready for the next step of the reaction sequence.

In general, the modified Jones oxidation procedure may conveniently be effected by reacting the alcohols (6) with pyridinium dichromate by contacting the reactants together in a water-trapping molecular sieve powder, e.g., a grounded 3 Angström molecular sieve), wherein said contact is in the presence of glacial acetic acid at about 0°C to 50°C, preferably at room temperature.

Alternatively, 1 to 5 equivalents of a chromic anhydride-pyridine complex (i.e., a Sarett reagent prepared *in situ* (see Fieser and Fieser "Reagents for Organic Synthesis" Vol. 1, pp. 145 and Sarett, et al., J.A.C.S. 25, 422, (1953)) said complex being prepared *in situ* in an inert solvent (e.g., CH₂Cl₂) in an inert atmosphere under anhydrous conditions at 0°C to 50°C to which complex is added 1 equivalent of the alcohols (6) allowing the reactants to interact for about 1 to 15 hours, followed by isolation of the desired product (7).

Another alternative process for converting the alcohols (6) to the desired ketones (7) is an oxidation reaction which employs periodane (i.e., 1,1,1-triacetoxy-2,1-benzoxiodol, (see Dess Martin, J. Org. Chem., 48, 4155, (1983)). This oxidation is effected by contacting about 1 equivalent of the alcohols (6) with 1 to 5 equivalents of periodane (preferably 1.5 equivalents), said reagent being in suspension in an inert solvent (e.g., methylene chloride) under an inert atmosphere (preferably nitrogen) under anhydrous conditions at 0°C to 50°C (preferably room temperature) and allowing the reactants to interact for about 1 to 48 hours.

Following oxidation and isolation, the acids of Formula (8) may be prepared by saponification procedures well known in the art, such as reaction of the esters with lithium hydroxide in a dioxane/water solvent mixture.

The products of Formula (9) may be obtained by coupling the acids (8) with the appropriate amine, using standard peptide coupling procedures using such coupling agents as isobutylchloroformate (and others as described above) according to procedures well known in the art. Following the coupling, the amino protecting groups may be selectively removed and the esters may be converted to their acids using standard procedures well known in the art.

The compounds of Formula (4) may also be converted to the desired malonyl derivatives wherein n is zero (i.e., R₅ is deleted) by acylation and oxidation procedures similar to those described above to produce compounds (12) and (13) respectively.

Alternatively, compounds of Formula (16) may be prepared by the reaction of Scheme B which essentially involves subjecting the hydroxamic derivatives of Formula (4) to a nucleophilic attack by an acylation with β-vinyl anion synton according to the techniques of R.R. Schmidt and J. Talbiershyl [Angew. Chem. Int. Ed. Engl. Vol. 15 (1976) No 3, page 171], which entails reaction of a β-acylenamine anion (14) (formed by treatment of the corresponding β-acylenamine with t-butyl lithium at temperatures below -100°C) with the hydroxomic derivatives (4) to produce compounds (15) which, upon sequention treatment with trifluoroacetic acid and water, form the desired compounds of Formula (16).

The solid phase sequential procedure can be performed using established automated methods such as by use of an automated peptide synthesizer. In this procedure an amino protected amino acid is bound to a resin support at the carboxy terminal end, the amino acid is deprotected at the amino position at which a peptide linkage is desired, the amino group neutralized with a base and the next amino protected amino acid in the desired sequence is coupled in a peptide linkage. The deprotection, neutralization and coupling steps are repeated until the desired polypeptide is synthesized. The compounds of the present invention are thus synthesized from their carboxy terminal end to their amino terminal end. The amino protected amino acid can be a conventional amino acid, a derivative or isomer thereof, or a spacer group. The resin support employed can be any suitable resin conventionally employed in the art for the solid phase preparation of polypeptides. The preferred resin is polystyrene which has been cross-linked with from about 0.5 to about 3% divinyl benzene, which has been either benzhydrylamidated, chloromethylated or hydroxymethylated to provide sites for amide or ester formation with the initially introduced amino protected amino acid.

An example of a hydroxymethyl resin is described by Bodansky et al. [Chem. Ind. (London) 38, 1597-98 (1966)]. The preparation of chloromethyl and benzhydrylamine resins are described by Stewart et al. ["Solid Phase Peptide Synthesis", 2nd Edition, Pierce Chemical Co., Rockford, Illinois (1984), Chapter 2, pp. 54-55]. Many of these resins are available commercially. In general, the amino protected amino acid which is desired on the carboxy-terminal end of the peptide is bound to the resin using standard procedures and practices as are well known and appreciated in the art. For example, the amino protected amino acid can be bound to the resin by the procedure of Gisin [Helv. Chem. Acta, 56, 1476 (1973)]. When it is desired to use a resin containing a benzhydrylamine moiety as the resin binding site an amino protected amino acid is coupled to the resin through an amide linkage between its α-carboxylic acid and the amino moiety of the resin. This coupling is effected using standard coupling procedures as described below. Many resin-bound amino acids are available commercially.

The α-amino protecting group employed with each amino acid introduced into the polypeptide sequence may be any such protecting group known in the art. Among the classes of amino protecting groups contemplated are: (1) acyl type protecting groups such as formyl, trifluoroacetyl, phthalyl, p-toluenesulfonyl (tosyl), benzenesulfonyl, nitrophenylsulfenyl, tritylsulfenyl, o-nitrophenoxyacetyl, and α-chlorobutyryl; (2) aromatic urethane type protecting groups such as benzyloxycarbonyl and substituted benzyloxycarbonyls such as p-chlorobenzyloxycarbonyl, p-methoxybenzyloxycarbonyl, p-nitrobenzyloxycarbonyl, p-bromobenzyloxycarbonyl, 1-(p-biphenylyl)-1-methylethoxycarbonyl, α-, α-dimethyl-3,5-dimethoxybenzyloxycarbonyl, and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl, and allyloxycarbonyl; (4) cycloalkyl urethane type protecting groups such as cyclopentyloxycarbonyl, adamantyloxycarbonyl, and cyclohexyloxycarbonyl; (5) thio urethane type protecting groups such as phenylthiocarbonyl; (6) alkyl type protecting groups such as triphenylmethyl (trityl) and benzyl (Bzl); (7) trialkylsilane protecting groups such as trimethylsilane. The preferred α-amino protecting group is tert-butyloxycarbonyl (Boc). The use of Boc as an α-amino protecting group for amino acids is described by Bodansky et al. in "The Practice of Peptide Synthesis", Springer-Verlag, Berlin (1984), p. 20.

Following the coupling of the amino protected amino acid to the resin support, the α-amino protecting group is removed using any suitable procedure such as by using trifluoroacetic acid, trifluoroacetic acid in dichloromethane, or HCl in dioxane. The deprotection is carried out at a temperature of between 0°C and room temperature. Other standard cleaving reagents may be used for removal of specific amino protecting groups under conditions well known and appreciated in the art.

After removal and neutralization of the α-amino protecting group the next desired amino-protected amino acid is coupled through a peptide linkage. This deprotection, neutralization and coupling procedure is repeated until a polypeptide of the desired sequence is obtained. Alternatively, multiple amino acid groups may be coupled by the solution method prior to coupling with the resin supported amino acid sequence.

The selection and use of an appropriate coupling reagent is within the skill of the ordinary practitioner in the art. Particularly suitable coupling reagents where the amino acid to be added is Gln, Asn, or Arg are N,N-dicyclohexylcarbodiimide and 1-hydroxybenzotriazole. The use of these reagents prevents nitrile and lactam formation. Other coupling agents are (1) carbodiimides (e.g., N,N-dicyclohexylcarbodiimide and N-ethyl-N'-(γ-dimethylaminopropylcarbodiimide); (3) ketenimines; (4) isoxazolium salts (e.g., N-ethyl-5-phenylisoxazolium-3-sulfonate); (5) monocyclic nitrogen containing heterocyclic amides of aromatic character containing one through four nitrogens in the ring such as imidazolides, pyrazolides, and 1,2,4-triazolides (specific heterocyclic amides that are useful include N,N-carbonyldiimidazole and N,N-carbonyl-di-1,2,4-triazole); (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-o-Ala-Boc); (8) nitrogen containing heterocyclic compounds having a hydroxy group on one ring nitrogen (e.g., N-hydroxyphthalimide, N-hydroxysuccinimide, and 1-hydroxybenzotriazole). Other activating reagents and their use in peptide coupling are described by Kapoor [J. Pharm. Sci., 59, 1-27 (1970)]. The generally preferred coupling method for the amino acids used in the present invention is the use of the symmetrical anhydride as the coupling agent.

The preferred coupling method for Gln, Asn and Arg is to react the protected amino acid, or derivatives or isomers thereof, with N,N-dicyclohexylcarbodiimide and 1-hydroxybenzotriazole (1:1) in N,N-dimethylformamide (DMF) in the presence of the resin or resin-bound amino acid or peptide. The preferred coupling method for other amino acids involves reacting the protected amino acid, or derivative or isomer thereof, with N,N-dicyclohexylcarbodiimide in dichloromethane to form the symmetrical anhydride. The symmetrical anhydride is then introduced into the solid phase reactor containing the resin or resin-bound amino acid or peptide, and the coupling is carried out in a medium of (DMF), or dichloromethane, or DMF: dichloromethane (1:1). A medium of DMF is preferred. The success of the coupling reaction at each stage of the synthesis is monitored by a ninhydrin test as described by Kaiser et al. [Analyt. Biochem. 34, 595 (1970)]. In cases where incomplete coupling occurs, the coupling procedure is repeated. If the coupling is still incomplete, the deprotected amine is capped with a suitable capping reagent to prevent its continued synthesis. Suitable capping reagents and the use thereof are well known and appreciated in the art. Examples of suitable capping reagents are acetic anhydride and acetylimidazole as described by Stewart et al. ["Solid Phase Peptide Synthesis", 2nd Ed., Pierce Chemical Co., Rockford, Ill. (1984), Chapter 2, p. 73].

After the desired amino acid sequence has been obtained, the peptide is cleaved from the resin. This can be effected by procedures which are well known and appreciated in the art, such as by hydrolysis of the ester or amide linkage to the resin. It is preferred to cleave the peptide from the benzhydrylamine resin with a solution of dimethyl sulfide, p-cresol, thiocresol, or anisole in anhydrous hydrogen fluoride. The cleavage reaction is preferably carried out at temperatures between about 0°C and about room temperature, and is allowed to continue preferably from between about 5 minutes to about 5 hours.

As is known in the art of solid phase peptide synthesis, many of the amino acids bear side chain functionalities requiring protection during the preparation of the peptide. The selection and use of an appropriate protecting group for these side chain functionalities is within the ability of those skilled in the art and will depend upon the amino acid to be protected and the presence of other protected amino acid residues in the peptide. The selection of such a side chain protecting group is critical in that it must not be removed during the deprotection and coupling steps of the synthesis. For example, when Boc is used as the α-amino protecting group, the following side chain protecting groups are suitable: p-toluenesulfonyl (tosyl) moieties can be used to protect the amino side chains of amino acids such as Lys and Arg; p-methylbenzyl, acetamidomethyl, benzyl (Bzl), or t-butylsulfonyl moieties can be used to protect the sulfide containing side chains of amino acids such as cysteine, homocysteine, penicillamine and the like or derivatives thereof; benzyl (Bzl) or cyclohexyl ester moieties can be used to protect carboxylic acid side chains of amino acids such as Asp, Glu; a benzyl (Bzl) ether can be used to protect the hydroxy containing side chains of amino acids such as Ser and Thr; and a 2-bromocarbobenzoxy (2Br-Z) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin.

The compounds are then isolated and purified by standard techniques. The desired amino acids, derivatives and isomers thereof can be obtained commercially or can be synthesized according to standard practices and procedures well known in the art.

The following specific examples are given to illustrate the preparation of this invention although the scope of compounds is meant to be limiting to the scope of compounds embraced by formula I.

### EXAMPLE 1

### 4-Hydroxy-6-phenyl-5-[((phenylmethoxy)carbonyl)amino]-3-oxohexanoic Acid Ethyl Ester

A solution of 2-hydroxy-4-phenyl-3-[((phenylmethoxy)carbonyl)amino]butanoic acid, N-methoxy-N-methylamide (372 mg, 1.0 mmol) in tetrahydrofuran is cooled to -78°C and ethyl lithioacetate (72 mg, 3.0 mmol) is added. The solution is stirred at -78°C for 1 hour, allowed to warm to room temperature, stirred for 1 hour and poured into dilute HCl. The product is extracted by ethyl acetate (3 x 150 ml) and the combined organic extracts are washed with NaHCO₃, dried over Na₂SO₄ and the solvent removed *in vacuo.* The crude product is purified by flash chromatography on silica gel.

### EXAMPLE 2

### 3,4-Dioxo-5-[((phenylmethoxy)carbonyl)amino]-6-phenylhexanoic Acid Ethyl Ester

A solution of 4-hydroxy-6-phenyl-5-[((phenylmethoxy)carbonyl)amino]-3-oxohexanoic acid ethyl ester (397 mg, 1.0 mmol) is dissolved in acetonitrile (15 ml) and the Dess-Martin periodinane (1.27 g, 3.0 mmol)) is added. To the mixture trifluoroacetic acid (342 mg, 3.0 mmol) is added and the mixture is stirred for 48 h. The solvent is removed *in vacuo* and EtOAc (100 ml) is added, followed by the addition of a solution of NaHCO₃ (0.80 g) and Na₂S₂O₃ (1.41 g) in H₂O (25 ml). The organic layer is separated and the aqueous phase extracted with ethyl acetate. The combined extracts are dried over Na₂S₂O₃ and the solvent is removed *in vacuo.* The product is purified by flash chromatography on silica gel.

### EXAMPLE 3

### 3,4-Dioxo-5-[((phenylmethoxy)carbonyl)amino]-6-phenylhexanoic Acid

To a solution of 3,4-dioxo-5-[((phenylmethoxy)carbonyl)amino]-6-phenylhexanoic acid ethyl ester (400 mg, 1.0 mmol) in dioxane/H₂O (10:1), lithium hydroxide (72 mg, 3.0 mmol) is added. The mixture is stirred for 3 h, the solvents are removed *in vacuo* and the crude product is used without purification.

### EXAMPLE 4

### N-[3,4-Dioxo-5-(((phenylmethoxy)carbonyl)amino))-6-phenylhexanoyl]glycinamide

To a solution of 3,4-dioxo-5-[((phenylmethoxy)carbonyl)amino]-6-phenylhexanoic acid (370 mg, 1.0 mmol) in methylene chloride (300 ml) is added N-methylmorpholine (0.30 g, 3.0 mmol). The mixture is cooled to -15°C, and isobutylchloroformate (136 mg, 1.0 mmol) is added. The mixture is stirred at -15°C for 15 minutes followed by the addition of N,O-dimethylhydroxylamine hydrochloride (194 mg, 1.0 mmol). The mixture is stirred at -15°C for 1 hour, allowed to warm to room temperature, and stirred for 3 h. The reaction mixture is poured into H₂O (300 ml), and the aqueous phase is extracted with methylene chloride (2 x 150 ml). The combined organic extracts are dried over Na₂SO₄, reduced in volume to 100 ml, and filtered through silica gel (2 in.). The solvent is removed *in vacuo* to give the crude product which is purified by flash chromatography.

The foregoing describes in detail the generic and specific aspects of the scope of the invention as well as the manner of making and using the invention. In addition thereto, although such procedures are known in the art, references setting forth state of the art procedures by which the compounds may be evaluated for their biochemical effects is also included herein.

For example, human elastase is assayed *in vitro* using chromophoric peptides, succinylalanylalanylalanyl-p-nitro-anilide, methoxysuccinylalanylalanylprolylvalyl-p-nitroanilide, and others, all of which are available commercially. The assay buffer, pH 8.0, and assay techniques are similar to those described by Lottenberg et al. Enzyme is purified from human sputum, although recently it has become commercially available. Kinetic characterization of immediate inhibitors is by means of the Dixon plot, whereas the characterization of slow- and/or tight-binding inhibitors used data analysis techniques reviewed by Williams and Morrison.

Similarly, the other proteases are assayed and effects of inhibitors are assessed *in vitro* by similar spectroscopic techniques: cathepsin G; thrombin; chymotrypsin; trypsin; plasmin; C1 esterase; urokinase; plasminogen activator; acrosin; β-lactamase; cathepsin B; pepsin; cathepsin D and leucine aminopeptidase. Pseudomonas elastase is measured in a coupled assay procedure using a human leastase substrate and microsomal aminopeptidase.

Radiometric assays of angiotensin I-converting enzyme and enkephalinase and their inhibitors are based on the procedure of Ryan and use tritiated substrate purchased from Ventrex Laboratories, Inc. Radioimmunoassay is used for studies with renin. C3-convertase is measured as described by Tack et al.

By following the technique referred to above, as well as by utilization of other known techniques, as well as by comparison with compounds known to be useful for treatment of the above-mentioned disease states, it is believed that adequate material is available to enable one of ordinary skill in the art to practice the invention. Of course, in the end-use application of the compounds of this invention, the compounds are preferably formulated into suitable pharmaceutical preparations such as tablets, capsules or elixers, for oral administration or in sterile solutions or suspensions for parenteral administration. The compounds of this invention can be administered to patients (animals and human) in need of such treatment in a dosage range of 0.01-10 mg per kg of body weight per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of Formula I or a physiologically acceptable salt is compounded with a physiologically acceptable vehicle, carrier, excipient, binder, perservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

## Claims (Claims for the following Contracting State(s): AT, BE, CH, DE, FR, GB, IT, LI, LU, NL, SE)

1. Compounds of the formula: 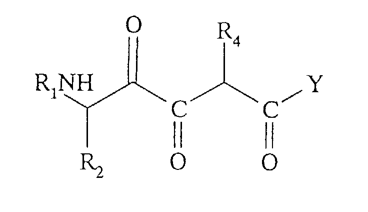 the hydrates, isosteres or the pharmaceutically acceptable salts thereof, wherein
R₁ is P₂P₃ or P₂P₃P_{g}, P_{g} being a Group K protecting group,
P₂ is an α-amino acid of Groups E or F,
P₃ is an α-amino acid of Groups B, E or F or is deleted,
R₂ is H, the side chain of an α-amino acid of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆ alkylene,
R₃ is C₁₋₆ alkyl, benzyl or phenethyl,
R₄ is the residue of an α-amino acid of Groups C, E or H, and
Y is OR₃ or NHR₃;
the said protecting group or α-amino acids of Groups B, C, E, F, J being as follows:
B: Glu, Asp
C: Ser, Thr, Gln, Asn, Cys, His, (3-pyrazolyl)Ala, (4-pyrimidinyl)Ala, and their N-methyl derivatives;
E: Ala, β-Ala, Leu, Ile, Val, n-Val, β-Val, Met, n-Leu and their methyl derivatives;
F: Phe, Tyr, O-Methyl Tyrosine, (3-pyrazolyl)Ala, (4-pyrimidinyl)Ala, Trp, Nal(1) and their N-methyl derivatives ;
J: 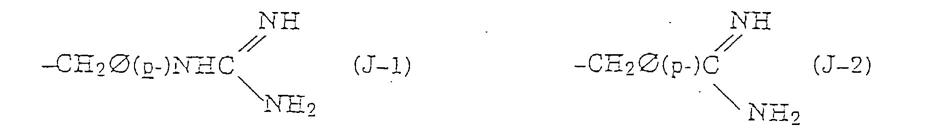 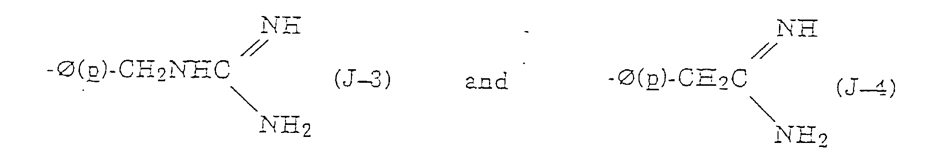 in which ⌀ represents a phenyl group ;
K: Acetyl (Ac); Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA).

2. Compound of claim 1, wherein R₁ is a protecting group CBZ, Bz or Ac.

3. Compound of claims 1 or 2, wherein P₂ is Val, Ile, Ala or Pro.

4. Compound of any one of claims 1 to 3, wherein P₃ is deleted or P₃ is Ile.

5. Compound of any one of claims 1 to 4, wherein R₂ is cyclohexylmethyl, naphthyl or Phe.

6. Compound of any one of claims 1 to 5, wherein R₃ is C₁-₆ alkyl.

7. A process for preparing compounds of the formula: 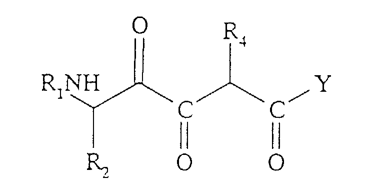 the hydrates, isosteres or the pharmaceutically accceptable salts thereof, wherein:
R1 is P₂P₃ or P₂P₃P_{g}, P_{g} being a Group K protecting group,
P₂ is an α-amino acid of Groups E or F,
P₃ is an α-amino acid of Groups B, E or F or is deleted;
R₂ is H, a residue of an α-amino acid of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆ alkylene,
R₃ is C₁₋₆ alkyl, benzyl or phenethyl,
R₄ is the residue of an α-amino acid of Groups C, E or H, and
Y is OR₃ or NHR₃,
the said protecting group or α-amino acids of Groups B, C, E, F, J being as follows:
B: Glu, Asp
C: Ser, Thr, Gln, Asn, Cys, His, (3-pyrazolyl)Ala, (4-pyrimidinyl)Ala, and their N-methyl derivatives;
E: Ala, β-Ala, Leu, Ile, Val, n-Val, β-Val, Met, n-Leu and their methyl derivatives;
F: Phe, Tyr, O-Methyl Tyrosine, (3-pyrazolyl)Ala, (4-pyrimidinyl)Ala, Trp, Nal(1) and their N-methyl derivatives ;
J: 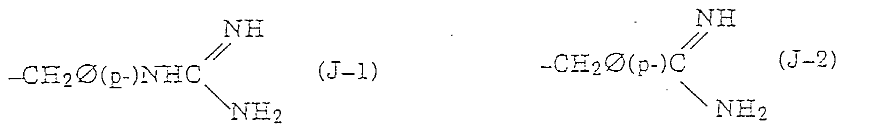 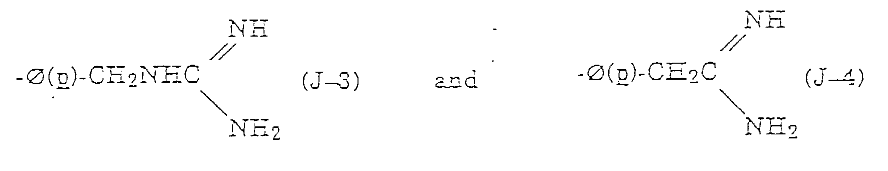 in which ⌀ represents a phenyl group ;
K: Acetyl (Ac); Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA),
said process comprising oxidation of a compound of the formula 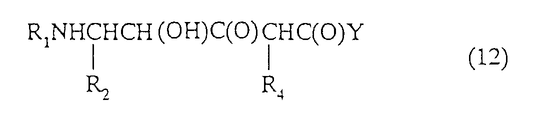 said oxidation being effected with:
(a) an *in situ*-formed sulfonium adduct formed by reaction of dimethylsulfoxide with (CF₃CO)₂O or (COCl)₂.
(b) a pyridinium dichromate in the presence of glacial acetic acid,
(c) an *in situ* chromic anhydride-pyridine complex, or (d) 1,1,1-triacetoxy-2, 1-benzoxiodol.

8. A compound according to any one of claims 1 to 6 for use as a pharmaceutically active compound.

## Claims (Claims for the following Contracting State(s): ES)

1. process for preparing compounds of the formula: 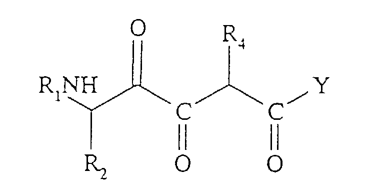 the hydrates, isosteres or the pharmaceutically accceptable salts thereof, wherein
R1 is P₂P₃ or P₂P₃P_{g}, P_{g} being a Group K protecting group,
P₂ is an α-amino acid of Groups E or F,
P₃ is an α-amino acid of Groups B, E or F or is deleted,
R₂ is H, a residue of an α-amino acid of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆ alkylene,
R₃ is C₁₋₆ alkyl, benzyl or phenethyl,
R₄ is the residue of an α-amino acid of Groups C, E or H, and
Y is OR₃ or NHR₃,
the said protecting group or α-amino acids of Groups B, C, E, F, J being as follows:
B: Glu, Asp;
C: Ser, Thr, Gln, Asn, Cys, His, (3-pyrazolyl)Ala, (4-pyrimidinyl)Ala, and their N-methyl derivatives;
E: Ala, β-Ala, Leu, Ile, Val, n-Val, β-Val, Met, n-Leu and their methyl derivatives;
F: Phe, Tyr, O-Methyl Tyrosine, (3-pyrazolyl)Ala, (4-pyrimidinyl)Ala, Trp, Nal(1) and their N-methyl derivatives ;
J: 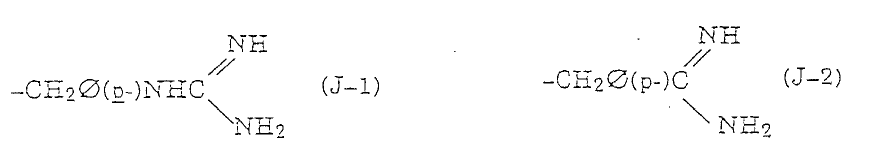 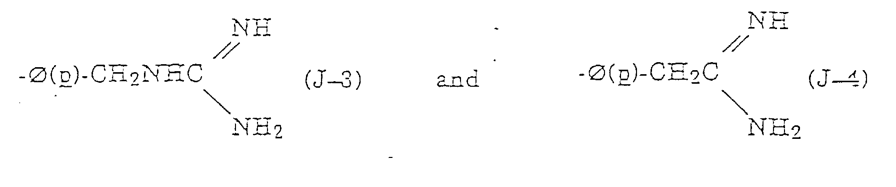 in which ⌀ represents a phenyl group ;
K: Acetyl (Ac); Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA) ;
said process comprises oxidation of a compound of the formula 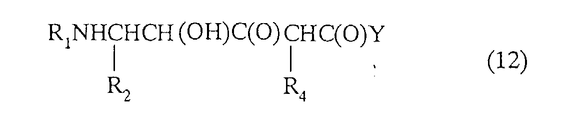 said oxidation being effected with:
(a) an *in situ*-formed sulfonium adduct formed by reaction of dimethylsulfoxide with (CF₃CO)₂O or (COCl)₂;
(b) a pyridinium dichromate in the presence of glacial acetic acid ;
(c) an *in situ* chromic anhydride-pyridine complex, or
(d) 1,1,1-triacetoxy-2, 1-benzoxiodol.

2. Process according to claim 1, wherein a compound of formula (12) in which R₁ is a protecting group CBZ, Bz or Ac is used.

3. Process according to claim 1, wherein a compound of formula (12) in which P₃ is deleted or P₃ is Ile is used.

4. Process according to claim 1, wherein a compound of formula (12) in which R₂ is cyclohexylmethyl, naphthyl or Phe is used.

5. Process according to claim 1, wherein a compound of formula (12) in which Y is OR₃ or NHR₃ in which R₃ is C₁-C₆ alkyl is used.

6. Use of a compound obtained according to the process of any one of claims 1 to 5 for the preparation of medicaments useful as calpain and cathepsin B inhibitors.

## Claims (Claims for the following Contracting State(s): GR)

1. Compounds of the formula: 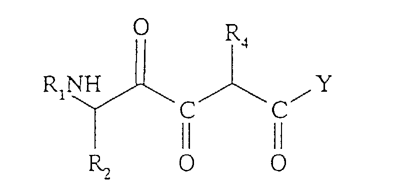 the hydrates, isosteres or the pharmaceutically acceptable salts thereof, wherein
R₁ is P₂P₃ or P₂P₃P_{g}, P_{g} being a Group K protecting group,
P₂ is an α-amino acid of Groups E or F,
P₃ is an α-amino acid of Groups B, E or F or is deleted,
R₂ is H, the side chain of an α-amino acid of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆, alkylene,
R₃ is C₁₋₆ alkyl, benzyl or phenethyl,
R₄ is the residue of an α-amino acid of Groups C, E or H, and
Y is OR₃ or NHR₃ ;
the said protecting group or α-amino acids of Groups B, C, E, F, J being as follows:
B: Glu, Asp
C: Ser, Thr, Gln, Asn, Cys, His, (3-pyrazolyl)Ala, (4-pyrimidinyl)Ala, and their N-methyl derivatives ; E: Ala, β-Ala, Leu, Ile, Val, n-Val, β-Val, Met, n-Leu and their methyl derivatives ;
F: Phe, Tyr, O-Methyl Tyrosine, (3-pyrazolyl)Ala, (4-pyrimidinyl)Ala, Trp, Nal(1) and their N-methyl derivatives ;
J: 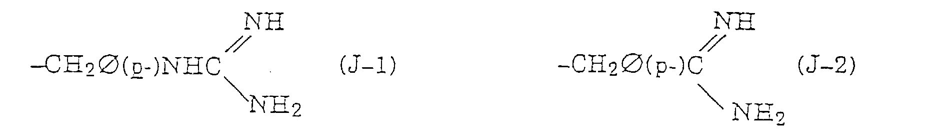 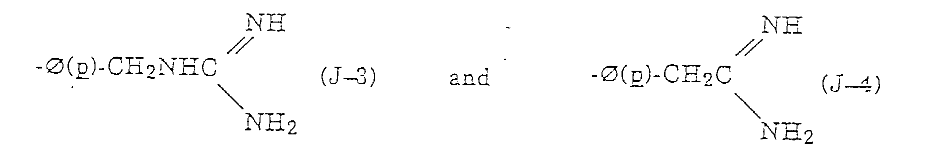 in which ⌀ represents a phenyl group ;
K: Acetyl (Ac) , Succinyl (Suc) , Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl -AdSO₂), 1-Adamantaneacetyl (AdAc), 2-Carboxybenzoyl (2-CBZ), Phenylacetyl, t-Butylacetyl (Tba), bis [(1-naphthyl)methyl]acetyl (BNMA).

2. Compound of claim 1, wherein R₁ is a protecting group CBZ, Bz or Ac.

3. Compound of claims 1 or 2, wherein P₂ is Val, Ile, Ala or Pro.

4. Compound of any one of claims 1 to 3, wherein P₃ is deleted or P₃ is Ile.

5. Compound of any one of claims 1 to 4, wherein R₂ is cyclohexylmethyl, naphthyl or Phe.

6. Compound of any one of claims 1 to 5, wherein R₃ is C₁-₆ alkyl.

7. A process for preparing compounds of the formula: 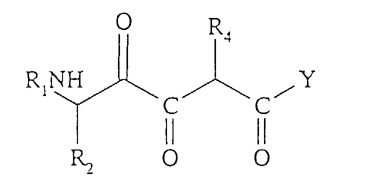 the hydrates, isosteres or the pharmaceutically accceptable salts thereof, wherein
R1 is P₂P₃ or P₂P₃P_{g}, P_{g} being a Group K protecting group,
P₂ is an α-amino acid of Groups E or F,
P₃ is an α-amino acid of Groups B, E or F or is deleted,
R₂ is H, a residue of an α-amino acid of Groups E, F, J, naphthyl, C₁₋₇ alkyl, benzyl, phenethyl, or A-SiR₇R₈R₉, R₇, R₈ and R₉ being C₁₋₁₀ alkyl, phenyl, benzyl, phenethyl and A is C₁₋₆ alkylene,
R₃ is C₁₋₆ alkyl, benzyl or phenethyl,
R₄ is the residue of an α-amino acid of Groups C, E or H, and
Y is OR₃ or NHR₃,
the said protecting group or α-amino acids of Groups B, C, E, F, J being as follows:
B: Glu, Asp
C: Ser, Thr, Gln, Asn, Cys, His, (3-pyrazolyl)Ala, (4-pyrimidinyl)Ala, and their N-methyl derivatives ; E: Ala, β-Ala, Leu, Ile, Val, n-Val, β-Val, Met, n-Leu and their methyl derivatives ;
F: Phe, Tyr, O-Methyl Tyrosine, (3-pyrazolyl)Ala, (4-pyrimidinyl)Ala, Trp, Nal(1), and their N-methyl derivatives ;
J: 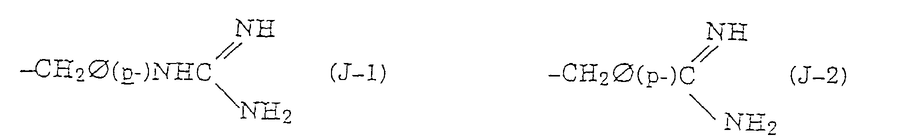 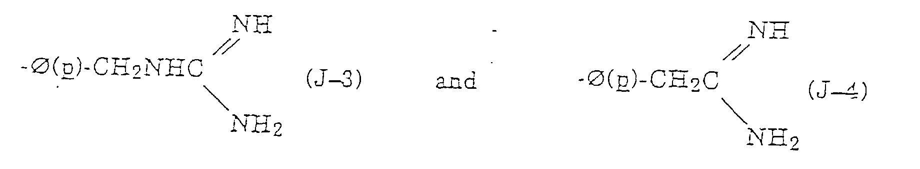 in which ⌀ represents a phenyl group ;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantanesulphonyl (AdSO₂), 1-Adamantaneacetyl (AdAc), 2-Carboxybenzoyl (2-CBZ), Phenylacetyl, t-Butylacetyl (Tba), bis [(l-naphthyl)methyl]acetyl (BNMA) ; said process comprises oxidation of a compound of the formula 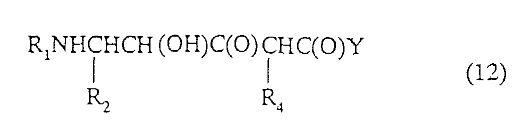
said oxidation being effected with
(a) an *in situ*-formed sulfonium adduct formed by reaction of dimethylsulfoxide with (CF₃CO)₂O or (COCl)₂.
(b) a pyridinium dichromate in the presence of glacial acetic acid,
(c) an *in situ* chromic anhydride-pyridine complex, or
(d) 1,1,1-triacetoxy-2, 1-benzoxiodol.

8. The use of the compounds according to claim 1 for the preparation of medicaments useful as calpain and cathepsin B inhibitors.

## Patentansprüche (Patentansprüche für folgende(n) Vertragsstaat(en): AT, BE, CH, DE, FR, GB, IT, LI, LU, NL, SE)

1. Verbindungen der Formel: 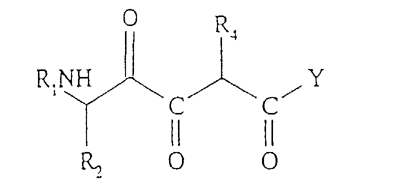 die Hydrate, Isostere oder die pharmazeutisch verträglichen Salze davon, wobei
R₁ P₂P₃ oder P₂P₃P_{g} ist, wobei P_{g} eine Schutzgruppe K ist;
P₂ eine α-Aminosäure der Gruppen E oder F ist,
P₃ eine α-Aminosäure der Gruppen B, E oder F ist oder nicht vorhanden ist;
R₂ H, die Seitenkette einer α-Aminosäure der Gruppen E, F, J, Naphthyl, C₁₋₇-Alkyl, Benzyl, Phenethyl oder A-Si-R₇R₈R₉ ist, wobei R₇, R₈ und R₉ die Bedeutung C₁₋₁₀-Alkyl, Phenyl, Benzyl, Phenethyl haben und A die Bedeutung C₁₋₆-Alkylen hat,
R₃ C₁₋₆-Alkyl, Benzyl oder Phenethyl ist,
R₄ der Rest einer α-Aminosäure der Gruppen C, E oder H ist und
Y OR₃ oder NHR₃ ist;
wobei die Schutzgruppe oder die α-Aminosäuren der Gruppen B, C, E, F, J die nachstehende Bedeutung haben:
B: Glu, Asp;
C: Ser, Thr, Gln, Asn, Cys, His, (3-Pyrazolyl)-Ala, (4-Pyrimidinyl)-Ala und ihre N-Methyl-Derivate;
E: Ala, β-Ala, Leu, Ile, Val, n-Val, β-Val, Met, n-Leu und ihre Methyl-Derivate;
F: Phe, Tyr, O-Methyl-Tyrosin, (3-Pyrazolyl)-Ala, (4-Pyrimidinyl)-Ala, Trp, Nal(1) und ihre N-Methyl-Derivate;
J: 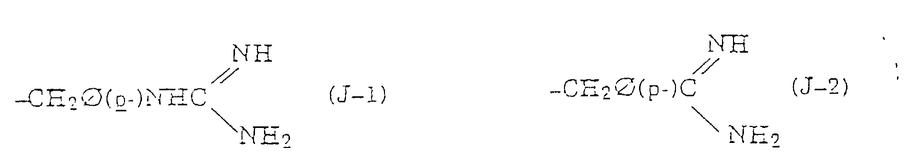 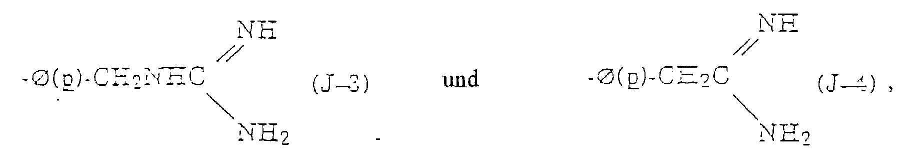 wobei ⌀ eine Phenylgruppe darstellt;
K: Acetyl (Ac), Succinyl. (Suc), Methoxysuccinyl (H3COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA).

2. Verbindung nach Anspruch 1, wobei R₁ eine Schutzgruppe CBZ, Bz oder Ac ist.

3. Verbindung nach einem der Ansprüche 1 oder 2, wobei P₂ Val, Ile, Ala oder Pro ist.

4. Verbindung nach einem der Ansprüche 1 bis 3, wobei P₃ nicht vorhanden ist oder P₃ Ile ist.

5. Verbindung nach einem der Ansprüche 1 bis 4, wobei R₂ Cyclohexylmethyl, Naphthyl oder Phe ist.

6. Verbindung nach einem der Ansprüche 1 bis 5, wobei R₃ C₁₋₆-Alkyl ist.

7. Verfahren zur Herstellung von Verbindungen der Formel: 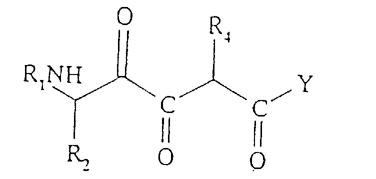 der Hydrate, Isostere oder der pharmazeutisch verträglichen Salze davon, wobei
R₁ P₂P₃ oder P₂P₃P_{g} ist, wobei P_{g} eine Schutzgruppe K ist;
P₂ eine α-Aminosäure der Gruppen E oder F ist,
P₃ eine α-Aminosäure der Gruppen B, E oder F ist oder nicht vorhanden ist,
R₂ H, ein Rest einer α-Aminosäure der Gruppen E, F, J, Naphthyl, C₁₋₇-Alkyl, Benzyl Phenethyl oder A-Si-R₇R₈R₉ ist, wobei R₇, R₈ und R₉ die Bedeutung C₁₋₁₀-Alkyl, Phenyl, Benzyl, Phenethyl haben und A die Bedeutung C₁₋₆-Alkylen hat,
R₃ C₁₋₆-Alkyl, Benzyl oder Phenethyl ist,
R₄ der Rest einer α-Aminosäure der Gruppen C, E oder H ist und
Y OR₃ oder NHR₃ ist;
wobei die Schutzgruppe oder die α-Aminosäuren der Gruppen B, C, E, F, J die nachstehende Bedeutung haben:
B: Glu, Asp;
C: Ser, Thr, Gln, Asn; Cys, His, (3-Pyrazolyl)-Ala, (4-Pyrimidinyl)-Ala und ihre N-Methyl-Derivate;
E: Ala, β-Ala, Leu, Ile, Val, n-Val, β-Val, Met, n-Leu und ihre Methyl-Derivate;
F: Phe, Tyr, O-Methyl-Tyrosin, (3-Pyrazolyl)-Ala, (4-Pyrimidinyl)-Ala, Trp, Nal(1) und ihre N-Methyl-Derivate;
J: 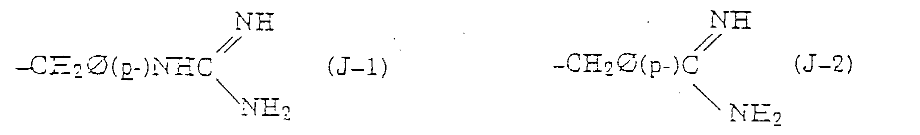 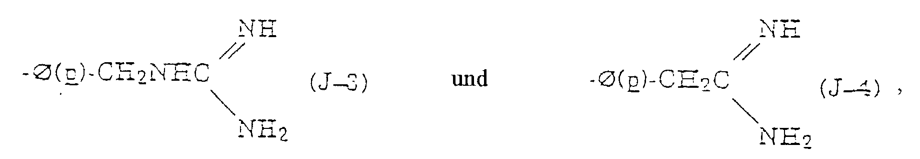 wobei ⌀ eine Phenylgruppe darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA), wobei das Verfahren die Oxidation einer Verbindung der Formel 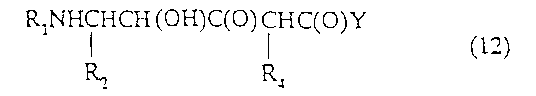
umfaßt, wobei die Oxidation durchgeführt wird mit:
(a) einem in situ hergestellten Sulfonium-Addukt, das durch Umsetzung von Dimethylsulfoxid mit (CF₃CO)₂O oder (COCl)₂ hergestellt wird,
(b) einem Pyridiniumdichromat in Gegenwart von Eisessig,
(c) einem in situ-Cbromsäureanhydrid-Pyridin-Komplex oder
(d) 1,1,1-Triacetoxy-2, 1-benzoxiodol.

8. Verbindung nach einem der Ansprüche 1 bis 6 zur Verwendung als eine pharmazeutisch aktive Verbindung.

## Patentansprüche (Patentansprüche für folgende(n) Vertragsstaat(en): ES)

1. Verfahren zur Herstellung von Verbindungen der Formel: 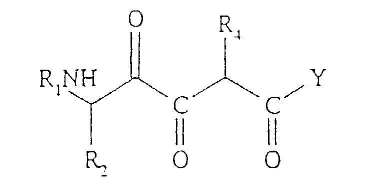 der Hydrate, Isostere oder der pharmazeutisch verträglichen Salze davon, wobei
R₁ P₂P₃ oder P₂P₃P_{g} ist, wobei P_{g} eine Schutzgruppe K ist;
P₂ eine α-Aminosäure der Gruppen E oder F ist,
P₃ eine α-Aminosäure der Gruppen B, E oder F ist oder nicht vorhanden ist,
R₂ H, ein Rest einer α-Aminosäure der Gruppen E, F, J, Naphthyl, C₁₋₇-Alkyl, Benzyl, Phenethyl oder A-Si-R₇R₈R₉ ist, wobei R₇, R₈ und R₉ die Bedeutung C₁₋₁₀-Alkyl, Phenyl, Benzyl, Phenethyl haben und A die Bedeutung C₁₋₆-Alkylen hat,
R₃ C₁₋₆-Alkyl, Benzyl oder Phenethyl ist,
R₄ der Rest einer α-Aminosäure der Gruppen C, E oder H ist und
Y OR₃ oder NHR₃ ist;
wobei die Schutzgruppe oder die α-Aminosäuren der Gruppen B, C, E, F, J die nachstehende Bedeutung haben:
B: Glu, Asp;
C: Ser, Thr, Gln, Asn, Cys, His, (3-Pyrazolyl)-Ala, (4-Pyrimidinyl)-Ala und ihre N-Methyl-Derivate;
E: Ala, β-Ala, Leu, Ile, Val, n-Val, β-Val, Met, n-Leu und ihre Methyl-Derivate;
F: Phe, Tyr, O-Methyl-Tyrosin, (3-Pyrazolyl)-Ala, (4-Pyrimidinyl)-Ala, Trp, Nal(l) und ihre N-Methyl-Derivate;
J: 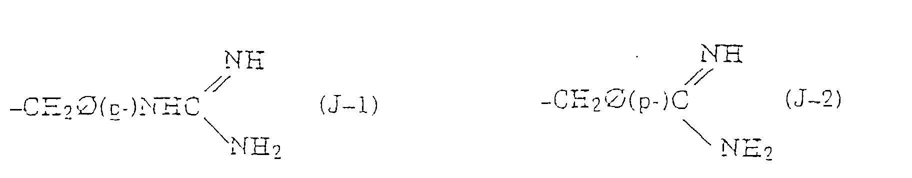 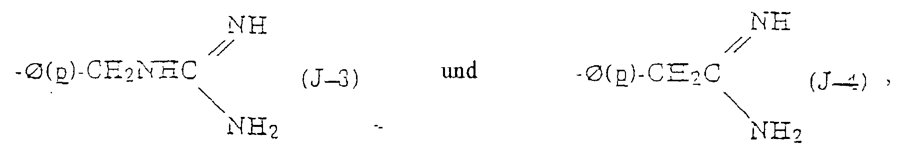 wobei ⌀ eine Phenylgruppe darstellt;
K: Acetyl (Ac), Succinyl (Suc), Methoxysuccinyl (H3COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA), wobei das Verfahren die Oxidation einer Verbindung der Formel 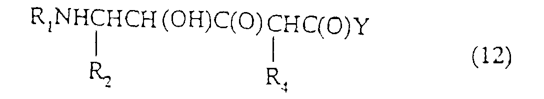
umfaßt, wobei die Oxidation durchgeführt wird mit:
(a) einem in situ hergestellten Sulfonium-Addukt, das durch Umsetzung von Dimethylsulfoxid mit (CF₃CO)₂O oder (COCl)₂ hergestellt wird,
(b) einem Pyridiniumdichromat in Gegenwart von Eisessig,
(c) einem in situ-Chromsäureanhydrid-Pyridin-Komplex oder
(d) 1,1,1-Triacetoxy-2,1-benzoxiodol.

2. Verfahren nach Anspruch 1, wobei eine Verbindung der Formel (12), in der R₁ eine Schutzgruppe CBZ, Bz oder Ac ist, verwendet wird.

3. Verfahren nach Anspruch 1, wobei eine Verbindung der Formel (12), in der P₃ nicht vorhanden ist oder P₃ Ile ist, verwendet wird.

4. Verfahren nach Anspruch 1, wobei eine Verbindung der Formel (12), in der R₂ Cyclohexylmethyl, Naphthyl oder Phe ist, verwendet wird.

5. Verfahren nach Anspruch 1, wobei eine Verbindung der Formel (12), in der Y OR₃ oder NHR₃ ist und in der R₃ C₁₋₆-Akyl ist, verwendet wird.

6. Verwendung einer nach dem Verfahren nach einem der Ansprüche 1 bis 5 erhaltenen Verbindung zur Herstellung von Medikamenten, die als Hemmstoffe von Calpain und Cathepsin B geeignet sind.

## Patentansprüche (Patentansprüche für folgende(n) Vertragsstaat(en): GR)

1. Verbindungen der Formel: 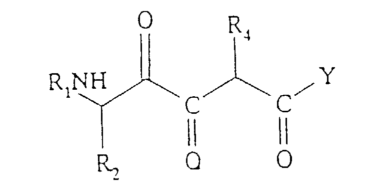 die Hydrate, Isostere oder die pharmazeutisch verträglichen Salze davon, wobei
R₁ P₂P₃ oder P₂P₃P_{g} ist, wobei P_{g} eine Schutzgruppe K ist;
P₂ eine α-Aminosäure der Gruppen E oder F ist,
P₃ eine α-Aminosäure der Gruppen B, E oder F ist oder nicht vorhanden ist,
R₂ H, die Seitenkette einer α-Aminosäure der Gruppen E, F, J, Naphthyl, C₁₋₇-Alkyl, Benzyl, Phenethyl oder A-Si-R₇R₈R₉ ist, wobei R₇, R₈ und R₉ die Bedeutung C₁₋₁₀-Alkyl, Phenyl, Benzyl, Phenethyl haben und A die Bedeutung C₁₋₆-Alkylen hat,
R₃ C₁₋₆-Alkyl, Benzyl oder Phenethyl ist,
R₄ der Rest einer α-Aminosäure der Gruppen C, E oder H ist und
Y OR₃ oder NHR₃ ist;
wobei die Schutzgruppe oder die α-Aminosäuren der Gruppen B, C, E, F, J die nachstehende Bedeutung haben:
B: Glu, Asp;
C: Ser, Thr, Gln, Asn, Cys, His, (3-pyrazolyl)-Ala, (4-Pyrimidinyl)-Ala und ihre N-Methyl-Derivate;
E: Ala, β-Ala, Leu, Ile, Val, n-Val, β-Val, Met, n-Leu und ihre Methyl-Derivate;
F: Phe, Tyr, O-Methyl-Tyrosin, (3-pyrazolyl)-Ala, (4-Pyrimidinyl)-Ala, Trp, Nal(1) und ihre N-Methyl-Derivate;
J: 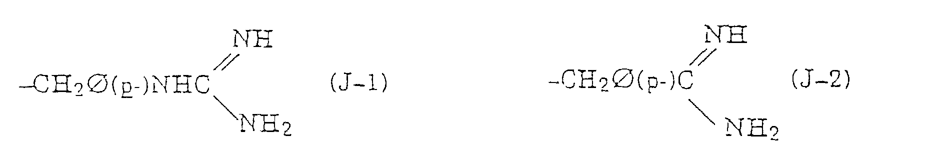 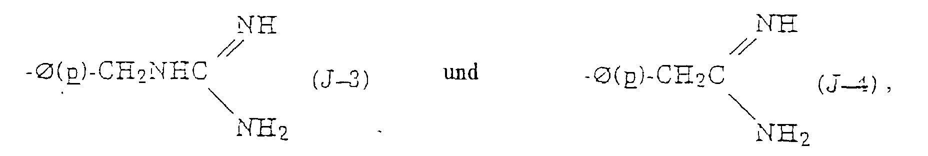 wobei ⌀ eine Phenylgruppe darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA).

2. Verbindung nach Anspruch 1, wobei R₁ eine Schutzgruppe CBZ, Bz oder Ac ist.

3. Verbindung nach einem der Ansprüche 1 oder 2, wobei P₂ Val, Ile, Ala oder Pro ist.

4. Verbindung nach einem der Ansprüche 1 bis 3, wobei P₃ nicht vorhanden ist oder P₃ Ile ist.

5. Verbindung nach einem der Ansprüche 1 bis 4, wobei R₂ Cyclohexylmethyl, Naphthyl oder Phe ist.

6. Verbindung nach einem der Ansprüche 1 bis 5, wobei R₃ C₁₋₆-Alkyl ist.

7. Verfahren zur Herstellung von Verbindungen der Formel: 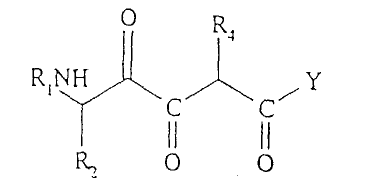 der Hydrate, Isostere oder der pharmazeutisch verträglichen Salze davon, wobei
R₁ P₂P₃ oder P₂P₃P_{g} ist, wobei P_{g} eine Schutzgruppe K ist;
P₂ eine α-Aminosäure der Gruppen E oder F ist,
P₃ eine α-Aminosäure der Gruppen B, E oder F ist oder nicht vorhanden ist,
R₂ H, ein Rest einer α-Aminosäure der Gruppen E, F, J, Naphthyl, C₁₋₇-Alkyl, Benzyl, Phenethyl oder A-Si-R₇R₈R₉ ist, wobei R₇, R₈ und R₉ die Bedeutung C₁₋₁₀-Alkyl, Phenyl, Benzyl, Phenethyl haben und A die Bedeutung C₁₋₆-Alkylen hat,
R₃ C₁₋₆-Alkyl, Benzyl oder Phenethyl ist,
R₄ der Rest einer α-Aminosäure der Gruppen C, E oder H ist und
Y OR₃ oder NHR₃ ist;
wobei die Schutzgruppe oder die α-Aminosäuren der Gruppen B, C, E, F, J die nachstehende Bedeutung haben:
B: Glu, Asp;
C: Ser, Thr, Gln, Asn, Cys, His, (3-Pyrazolyl)-Ala, (4-Pyrimidinyl)-Ala und ihre N-Methyl-Derivate;
E: Ala, β-Ala, Leu, Ile, Val, n-Val, β-Val, Met, n-Leu und ihre Methyl-Derivate;
F: Phe, Tyr, O-Methyl-Tyrosin, (3-Pyrazolyl)-Ala, (4-Pyrimidinyl)-Ala, Trp, Nal(1) und ihre N-Methyl-Derivate;
J: 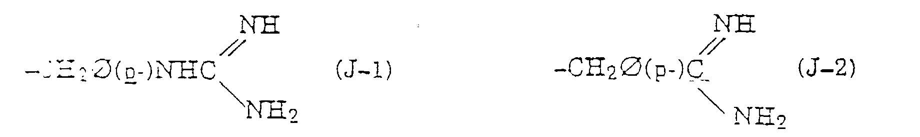 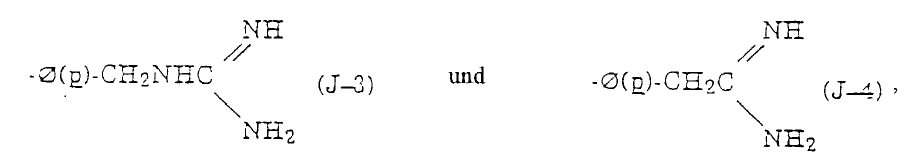 wobei ⌀ eine Phenylgruppe darstellt;
K: Acetyl (Ac), Succinyl (Suc), Methoxysuccinyl (H₃COSuc), Benzoyl (Bz), t-Butyloxycarbonyl (Boc), Carbobenzoxy (CBZ), Tosyl (Ts), Dansyl (DNS), Isovaleryl (Iva), Methoxysuccinyl (MeOSuc), 1-Adamantansulfonyl (AdSO₂), 1-Adamantanacetyl (AdAc), 2-Carboxybenzoyl (2-CBZ), Phenylacetyl, t-Butylacetyl (Tba), Bis[(1-naphthyl)methyl]acetyl (BNMA), wobei das Verfahren die Oxidation einer Verbindung der Formel 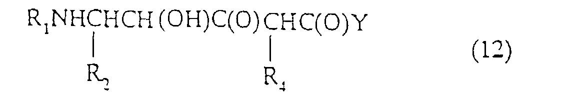
umfaßt, wobei die Oxidation durchgeführt wird mit:
(a) einem in situ hergestellten Sulfonium-Addukt, das durch Umsetzung von Dimethylsulfoxid mit (CF₃CO)₂O oder (COCl)₂ hergestellt wird,
(b) einem Pyridiniumdichromat in Gegenwart von Eisessig,
(c) einem in situ-Chromsäureanhydrid-Pyridin-Komplex oder
(d) 1,1,1-Triacetoxy-2,1-benzoxiodol.

8. Verwendung von Verbindungen nach Anspruch 1 zur Herstellung von Medikamenten, die als Hemmstoffe von Calpain und Cathepsin B geeignet sind.

## Revendications (Revendications pour l'(les) Etat(s) contractant(s) suivant(s): AT, BE, CH, DE, FR, GB, IT, LI, LU, NL, SE)

1. Composés de formule : 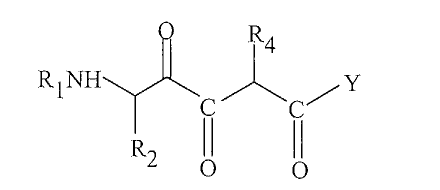 leurs hydrates, isostères ou sels pharmaceutiquement acceptables, où
R₁ est P₂P₃ ou P₂P₃P_{g}, P_{g} étant un groupe protecteur du Groupe K,
P₂ est un acide α-aminé des Groupes E ou F,
P₃ est un acide α-aminé des Groupes B, E, F ou est supprimé,
R₂ est H, la chaîne latérale d'un acide α-aminé des Groupes E, F, J, un naphtyle, un alkyle en C₁-C₇, un benzyle, un phénéthyle ou -A-SiR₇R₈R₉, R₇, R₈ et R₉ étant un alkyle en C₁-C₁₀, un phényle, un benzyle, un phénéthyle et A est un alkylène en C₁-C₆,
R₃ est un alkyle en C₁-C₆, un benzyle ou un phénétyle,
R₄ est le résidu d'un acide α-aminé des Groupes C, E ou H, et
Y est OR₃ ou NHR₃,
ledit groupe protecteur ou les acides α-aminés des Groupes B, C, E, F, J étant comme suit :
B : Glu, Asp ;
C : Ser, Thr, Gln, Asn, Cys, His, (3-pyrazolyl)Ala, (4-pyrimidinyl)Ala et leurs dérivés N-méthylés ;
E : Ala, β-Ala, Leu, Ile, Val, n-Val, β-Val, Met, n-Leu et leurs dérivés méthylés ;
F: Phe, Tyr, O-méthyltyrosine, (3-pyrazolyl)Ala, (4-pyrimidinyl)Ala, Trp, Nal(1) et leurs dérivés N-méthylés ;
J: 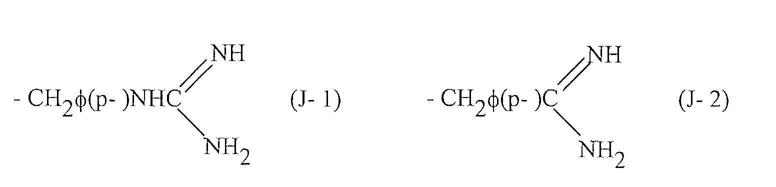 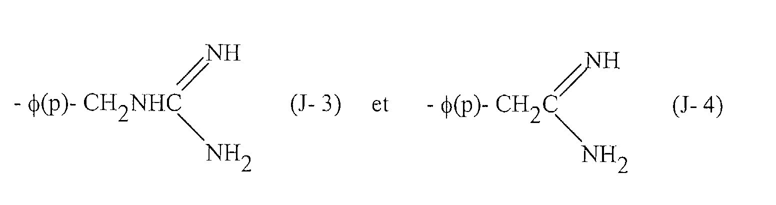 où φ représente un groupe phényle ;
K: Acétyle (Ac), Succinyle (Suc), Méthoxysuccinyle (H₃COSuc), Benzoyle (Bz), t-butyloxycarbonyle (Boc), Carbobenzoxy (CBZ), Tosyle (Ts), Dansyle (DNS), Isovaléryle (Iva), Méthoxysuccinyle (MeOSuc), 1 -adamantanesulfonyle (AdSO₂), 1-adamantaneacétyle (AdAc), 2-carboxybenzoyle (2-CBZ), phénylacétyle, t-butylacétyle (Tba), bis[(1-naphtyl)méthyl]acétyle (BNMA).

2. Composé selon la revendication 1, dans lequel R₁ est un groupe protecteur CBZ, Bz ou Ac.

3. Composé selon les revendications 1 ou 2, dans lequel P₂ est Val, Ile, Ala ou Pro.

4. Composé selon l'une quelconque des revendications 1 à 3, dans lequel P₃ est supprimé ou P₃ est Ile.

5. Composé selon l'une quelconque des revendications 1 à 4, dans lequel R₂ est un cyclohexylméthyle, un naphtyle ou Phe.

6. Composé selon l'une quelconque des revendications 1 à 5, dans lequel R₃ est un alkyle en C₁-C₆.

7. Procédé de préparation de composés de formule : 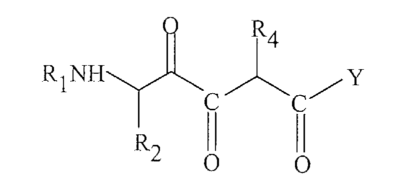 de leurs hydrates, isostères ou sels pharmaceutiquement acceptables, où
R₁ est P₂P₃ ou P₂P₃P_{g}, P_{g} étant un groupe protecteur du Groupe K,
P₂ est un acide α-aminé des Groupes E ou F,
P₃ est un acide α-aminé des Groupes B, E, F ou est supprimé,
R₂ est H, un résidu d'un acide α-aminé des Groupes E, F, J, un naphtyle, un alkyle en C₁-C₇, un benzyle, un phénéthyle ou A-SiR₇R₈R₉, R₇, R₈ et R₉ étant un alkyle en C₁-C₁₀, un phényle, un benzyle, un phénéthyle et A est un alkylène en C₁-C₆,
R₃ est un alkyle en C₁-C₆, un benzyle ou un phénéthyle,
R₄ est le résidu d'un acide α-aminé des Groupes C, E ou H, et
Y est OR₃ ou NHR₃,
ledit groupe protecteur ou les acides α-aminés des Groupes B, C, E, F, J étant comme suit :
B : Glu, Asp ;
C : Ser, Thr, Gln, Asn, Cys, His, (3-pyrazolyl)Ala, (4-pyrimidinyl)Ala et leurs dérivés N-méthylés ;
E : Ala, β-Ala, Leu, Ile, Val, n-Val, β-Val, Met, n-Leu et leurs dérivés méthylés ;
F: Phe, Tyr, O-méthyltyrosine, (3-pyrazolyl)Ala, (4-pyrimidinyl)Ala, Trp, Nal(1) et leurs dérivés N-méthylés ;
J : 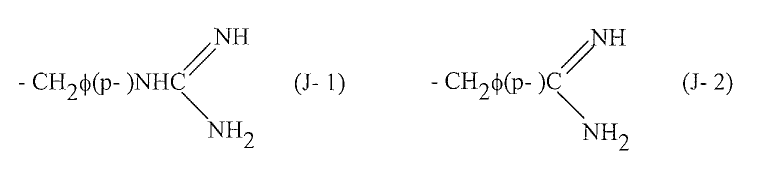 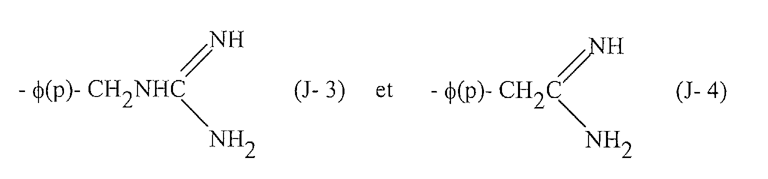 où φ représente un groupe phényle ;
K: Acétyle (Ac), Succinyle (Suc), Méthoxysuccinyle (H₃COSuc), Benzoyle (Bz), t-butyloxycarbonyle (Boc), Carbobenzoxy (CBZ), Tosyle (Ts), Dansyle (DNS), Isovaléryle (Iva), Méthoxysuccinyle (MeOSuc), 1-adamantanesulfonyle (AdSO₂), 1-adamantaneacétyle (AdAc), 2-carboxybenzoyle (2-CBZ), Phénylacétyle, t-butylacétyle (Tba), bis [(1-naphtyl)méthyl]acétyle(BNMA),
ledit procédé comprenant l'oxydation d'un composé de formule : 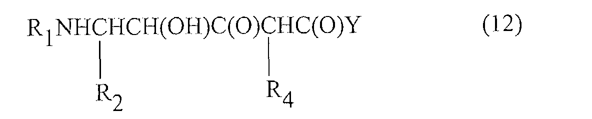 ladite oxydation étant réalisée avec :
(a) un produit d'addition de sulfonium formé *in situ,* formé par la réaction de diméthylsulfoxyde avec (CF₃CO)₂O ou (COCl)₂,
(b) un dichromate de pyridinium en présence d'acide acétique glacial,
(c) un complexe anhydride de chrome-pyridine *in situ* ou
(d) du 1,1,1-triacétoxy-2,1-benzoxiodol.

8. Un composé selon l'une quelconque des revendications 1 à 6, pour son utilisation en tant que composé pharmaceutiquement actif.

## Revendications (Revendications pour l'(les) Etat(s) contractant(s) suivant(s): ES)

1. Procédé de préparation de composés de formule : 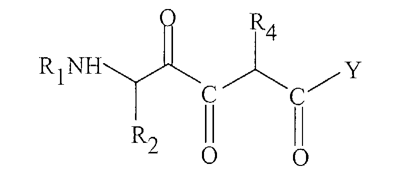 de leurs hydrates, isostères ou sels pharmaceutiquement acceptables, où
R₁ est P₂P₃ ou P₂P₃P_{g}, P_{g} étant un groupe protecteur du Groupe K,
P₂ est un acide α-aminé des Groupes E ou F,
P₃ est un acide α-aminé des Groupes B, E, F ou est supprimé,
R₂ est H, un résidu d'un acide α-aminé des Groupes E, F, J, un naphtyle, un alkyle en C₁-C₇, un benzyle, un phénéthyle ou A-SiR₇R₈R₉, R₇, R₈ et R₉ étant un alkyle en C₁-C₁₀, un phényle, un benzyle, un phénéthyle et A est un alkylène en C₁-C₆,
R₃ est un alkyle en C₁-C₆, un benzyle ou un phénéthyle,
R₄ est le résidu d'un acide α-aminé des Groupes C, E ou H, et
Y est OR₃ ou NHR₃,
ledit groupe protecteur ou les acides α-aminés des Groupes B, C, E, F, J étant comme suit :
B: Glu, Asp ;
C : Ser, Thr, Gln, Asn, Cys, His, (3-pyrazolyl)Ala, (4-pyrimidinyl)Ala et leurs dérivés N-méthylés ;
E : Ala, β-Ala, Leu, Ile, Val, n-Val, β-Val, Met, n-Leu et leurs dérivés méthylés ;
F: Phe, Tyr, O-méthyltyrosine, (3-pyrazolyl)Ala, (4-pyrimidinyl)Ala, Trp, Nal(1) et leurs dérivés N-méthylés ;
J: 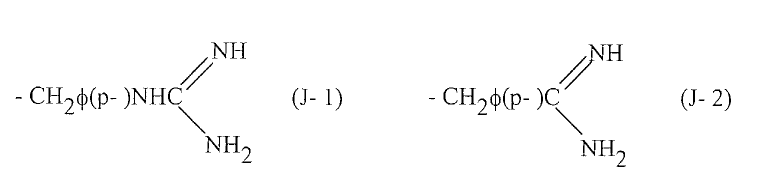 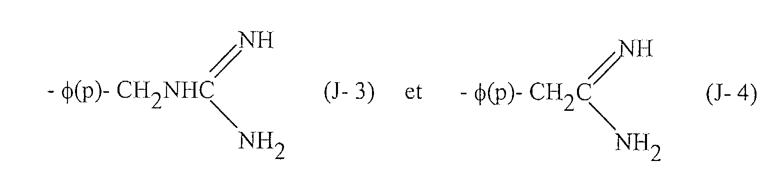 où φ représente un groupe phényle ;
K: Acétyle (Ac), Succinyle (Suc), Méthoxysuccinyle (H₃COSuc), Benzoyle (Bz), t-butyloxycarbonyle (Boc), Carbobenzoxy (CBZ), Tosyle (Ts), Dansyle (DNS), Isovaléryle (Iva), Méthoxysuccinyle (MeOSuc); 1-adamantanesulfonyle (AdSO₂), 1-adamantaneacétyle (AdAc), 2-carboxybenzoyle (2-CBZ), phénylacétyle, t-butylacétyle (Tba), bis[(1-naphtyl)méthyl] acétyle (BNMA),
ledit procédé comprenant l'oxydation d'un composé de formule : 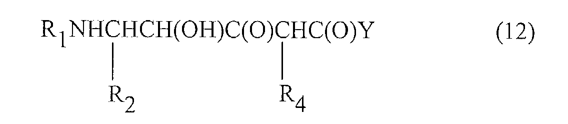 ladite oxydation étant réalisée avec :
(a) un produit d'addition de sulfonium formé *in situ* par la réaction de diméthylsulfoxyde avec (CF₃CO)₂O ou (COCl)₂,
(b) un dichromate de pyridinium en présence d'acide acétique glacial,
(c) un complexe anhydride de chrome-pyridine *in situ* ou
(d) du 1,1,1-triacétoxy-2,1-benzoxiodol.

2. Procédé selon la revendication 1, dans lequel on utilise un composé de formule (12) dans laquelle R₁ est un groupe protecteur CBZ, Bz ou Ac.

3. Procédé selon la revendication 1, dans lequel on utilise un composé de formule (12) dans laquelle P₃ est supprimé ou P₃ est Ile.

4. Procédé selon la revendication 1, dans lequel on utilise un composé de formule (12) dans laquelle R₂ est un cyclohexylméthyle, un naphtyle ou Phe.

5. Procédé selon la revendication 1, dans lequel on utilise un composé de formule (12) dans laquelle Y est OR₃ ou NHR₃, R₃ étant un alkyle en C₁-C₆.

6. Utilisation d'un composé obtenu par le procédé, selon l'une quelconque des revendications 1 à 5, pour la préparation de médicaments utiles en tant qu'inhibiteurs de la calpaine et de la cathepsine B.

## Revendications (Revendications pour l'(les) Etat(s) contractant(s) suivant(s): GR)

1. Composés de formule : 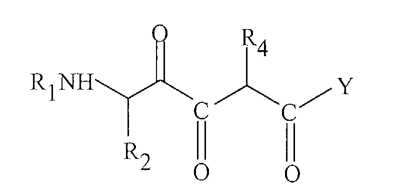 ses hydrates, isostères ou sels pharmaceutiquement acceptables, où
R₁ est P₂P₃ ou P₂P₃P_{g}, P_{g} étant un groupe protecteur du Groupe K,
P₂ est un acide α-aminé des Groupes E ou F,
P₃ est un acide α-aminé des Groupes B, E, F ou est supprimé,
R₂ est H, la chaîne latérale d'un acide α-aminé des Groupes E, F, J, un naphtyle, un alkyle en C₁-C₇, un benzyle, un phénéthyle ou A-SiR₇R₈R₉, R₇; R₈ et R₉ étant un alkyle en C₁-C₁₀, un phényle, un benzyle, un phénéthyle et A est un alkylène en C₁-C₆;
R₃ est un alkyle en C₁-C₆, un benzyle ou un phénéthyle,
R₄ est le résidu d'un acide α-aminé des Groupes C, E ou H, et
Y est OR₃ ou NHR₃,
ledit groupe protecteur ou les acides α-aminés des Groupes B, C, E, F, J étant comme suit :
B : Glu, Asp
C : Ser, Thr, Gln, Asn, Cys, His, (3-pyrazolyl)Ala, (4-pyrimidinyl)Ala et leurs dérivés N-méthylés
E: Ala, β-Ala, Leu, Ile, Val, n-Val, β-Val, Met, n-Leu et leurs dérivés méthylés
F: Phe, Tyr, O-méthyltyrosine, (3-pyrazolyl)Ala, (4-pyrimidinyl)Ala, Trp, Nal(1) et leurs dérivés N-méthylés
J: 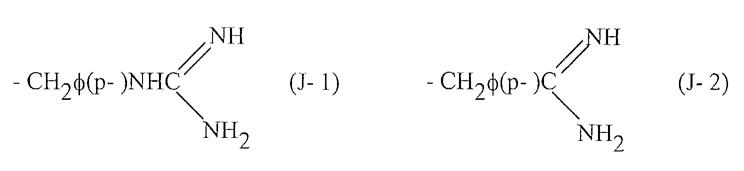 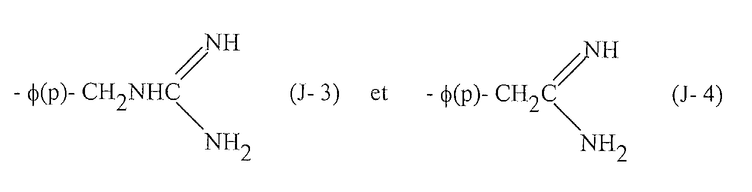 où φ représente un groupe phényle ;
K: Acétyle (Ac), Succinyle (Suc), Méthoxysuccinyle (H₃COSuc), Benzoyle (Bz), t-butyloxycarbonyle (Boc), Carbobenzoxy (CBZ), Tosyle (Ts), Dansyle (DNS), Isovaléryle (Iva), Méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis[(1-naphtyl)méthyl]acétyle (BNMA).

2. Composé selon la revendication 1, dans lequel R₁ est un groupe protecteur CBZ, Bz ou Ac.

3. Composé selon les revendications 1 ou 2, dans lequel P₂ est Val, Ile, Ala ou Pro.

4. Composé selon l'une quelconque des revendications 1 à 3, dans lequel P₃ est supprimé ou P₃ est Ile.

5. Composé selon l'une quelconque des revendications 1 à 4, dans lequel R₂ est un cyclohexylméthyle, un naphtyle ou Phe.

6. Composé selon l'une quelconque des revendications 1 à 5, dans lequel R₃ est un alkyle en C₁-C₆.

7. Procédé de préparation de composés de formule : 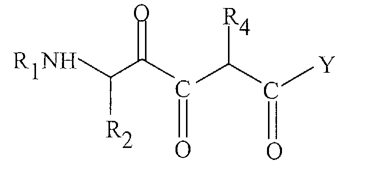 de leurs hydrates, isostères ou sels pharmaceutiquement acceptables, où
R₁ est P₂P₃ ou P₂P₃P_{g}, P_{g} étant un groupe protecteur du Groupe K,
P₂ est un acide α-aminé des Groupes E ou F,
P₃ est un acide α-aminé des Groupes B, E, F ou est supprimé,
R₂ est H, un résidu d'un acide α-aminé des Groupes E, F, J, un naphtyle, un alkyle en C₁-C₇, un benzyle, un phénéthyle ou A-SiR₇R₈R₉, R₇, R₈ et R₉ étant un alkyle en C₁-C₁₀, un phényle, un benzyle, un phénéthyle et A est un alkylène en C₁-C₆,
R₃ est un alkyle en C₁-C₆, un benzyle ou un phénéthyle,
R₄ est le résidu d'un acide α-aminé des Groupes C, E ou H, et
Y est OR₃ ou NHR₃,
ledit groupe protecteur ou les acides α-aminés des Groupes B, C, E, F, J étant comme suit :
B : Glu, Asp ;
C : Ser, Thr, Gln, Asn, Cys, His, (3-pyrazolyl)Ala, (4-pyrimidinyl)Ala et leurs dérivés N-méthylés ;
E: Ala, β-Ala, Leu, Ile, Val, n-Val, β-Val, Met, n-Leu et leurs dérivés méthylés;
F: Phe, Tyr, O-méthyltyrosine, (3-pyrazolyl)Ala, (4-pyrimidinyl)Ala, Trp, Nal(1) et leurs dérivés N-méthylés ;
J: 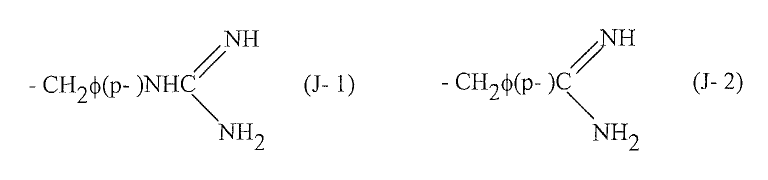 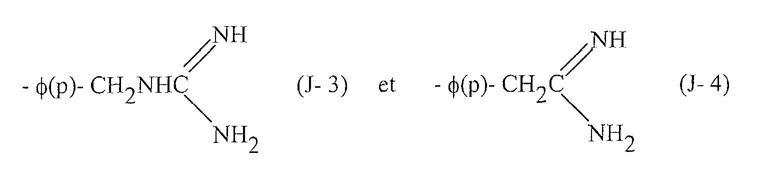 où φ représente un groupe phényle ;
K: Acétyle (Ac), Succinyle (Suc), Méthoxysuccinyle (H₃COSuc), Benzoyle (Bz), t-butyloxycarbonyle (Boc), Carbobenzoxy (CBZ), Tosyle (Ts), Dansyle (DNS), isovaléryle (Iva), Méthoxysuccinyle (MeOSuc), 1-adamantanesulfonyle (AdSO₂), 1-adamantaneacétyle (AdAc), 2-carboxybenzoyle (2-CBZ), phénylacétyle, t-butylacétyle (Tba), bis[(1-naphtyl)méthyl]acétyle (BNMA),
ledit procédé comprenant l'oxydation d'un composé de formule : 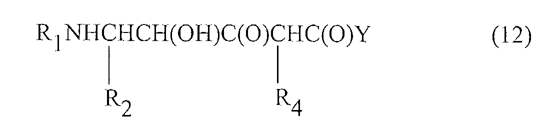 ladite oxydation étant réalisée avec :
(a) un produit d'addition de sulfonium formé *in situ* par la réaction de diméthylsulfoxyde avec (CF₃CO)₂O ou (COCl)₂,
(b) un dichromate de pyridinium en présence d'acide acétique glacial,
(c) un complexe anhydride de chrome-pyridine *in situ* ou
(d) du 1,1,1-triacétoxy-2,1-benzoxiodol.

8. Utilisation des composés selon la revendication 1 pour la préparation de médicaments utiles en tant qu'inhibiteurs de la calpaine et de la cathepsine B.
